# EUROPÄISCHE PATENTSCHRIFT

(11) **EP 2 467 478 B1**
(45) Veröffentlichungstag und Bekanntmachung des Hinweises auf die Patenterteilung: **16.09.2015**
(21) Anmeldenummer: 10757053.3
(22) Anmeldetag: 18.08.2010
(51) Int. Cl.: C12N 9/04

(54) **STABILISIERUNG VON ENZYMEN MIT STABILEN COENZYMEN**
Stabilisation of enzymes with stable coenzymes
Stabilisation d'enzymes à l'aide de coenzymes stables

(30) Priorität: 20.08.2009 EP 09168327
(43) Veröffentlichungstag der Anmeldung: 27.06.2012
(73) Patentinhaber: F.Hoffmann-La Roche AG, 4070 Basel (CH); Roche Diagnostics GmbH, 68305 Mannheim (DE)
(72) Erfinder: RÖDEL, Wolfgang, 69123 Heidelberg (DE); HORN, Carina, 68647 Biblis (DE); STEINKE, Nelli, 68623 Lampertheim (DE); BUCCI, Nadine, 69168 Wiesloch (DE); MEIER, Thomas, 81373 München (DE); SCHMUCK, Rainer, 83671 Benediktbeuern (DE); NAGEL, Rolf, 68642 Bürstadt (DE); HEINDL, Dieter, 82396 Paehl (DE)
(74) Vertreter: Weickmann & Weickmann
(86) Internationale Anmeldenummer: PCT/EP2010/062045
(87) Internationale Veröffentlichungsnummer: WO 2011/020856

(56) Entgegenhaltungen:
- EP-A- 2 093 284
- WO-A-97/19190
- WO-A-98/33936
- WO-A-2007/012494
- OSBORNE H H ET AL: "AN INVESTIGATION OF THE NAD INDUCED TIGHTENING OF THE STRUCTURE OF GLYCERALDEHYDE 3 PHOSPHATE DEHYDROGENASE EC-1.2.1.12" BIOCHEMICAL JOURNAL, Bd. 157, Nr. 1, 1976, Seiten 255-259, XP002553207 ISSN: 0264-6021
- ZHAO KEHAO ET AL: "Structural basis for nicotinamide cleavage and ADP-ribose transfer by NAD+-dependent Sir2 histone/protein deacetylases" PROCEEDINGS OF THE NATIONAL ACADEMY OF SCIENCES OF THE UNITED STATES OF AMERICA, Bd. 101, Nr. 23, 8. Juni 2004 (2004-06-08) , Seiten 8563-8568, XP002553208 ISSN: 0027-8424

### SEQUENCE LISTING

<110> F. Hoffmann - La Roche AG
   Roche Diagnostics GmbH
<120> Stabilisierung von Enzymen mit stabilen Coenzymen
<130> 46200P WO
<150> EP 09 168 327.6
   <151> 2009-08-20
<160> 2
<170> PatentIn version 3.3
<210> 1
   <211> 261
   <212> PRT
   <213> Artificial
<220>
   <223> Mutant of glucose dehydrogenase from Bacillus subtilis
<220>
   <221> MUTAGEN
   <222> (96)..(96)
<220>
   <221> MUTAGEN
   <222> (170)..(170)
<400> 1
   Met Tyr Pro Asp Leu Lys Gly Lys Val Val Ala Ile Thr Gly Ala Ala
   1               5                   10                  15
   Ser Gly Leu Gly Lys Ala Met Ala Ile Arg Phe Gly Lys Glu Gln Ala
               20                  25                  30
   Lys Val Val Ile Asn Tyr Tyr Ser Asn Lys Gln Asp Pro Asn Glu Val
           35                  40                  45
   Lys Glu Glu Val Ile Lys Ala Gly Gly Glu Ala Val Val Val Gln Gly
       50                  55                  60
   Asp Val Thr Lys Glu Glu Asp Val Lys Asn Ile Val Gln Thr Ala Ile
   65                  70                  75                  80
   Lys Glu Phe Gly Thr Leu Asp Ile Met Ile Asn Asn Ala Gly Leu Gly
                   85                  90                  95
   Asn Pro Val Pro Ser His Glu Met Pro Leu Lys Asp Trp Asp Lys Val
               100                 105                 110
   Ile Gly Thr Asn Leu Thr Gly Ala Phe Leu Gly Ser Arg Glu Ala Ile
           115                 120                 125
   Lys Tyr Phe Val Glu Asn Asp Ile Lys Gly Asn Val Ile Asn Met Ser
       130                 135                 140
   Ser Val His Glu Val Ile Pro Trp Pro Leu Phe Val His Tyr Ala Ala
   145                 150                 155                 160
   Ser Lys Gly Gly Ile Lys Leu Met Thr Lys Thr Leu Ala Leu Glu Tyr
                   165                 170                 175
   Ala Pro Lys Gly Ile Arg Val Asn Asn Ile Gly Pro Gly Ala Ile Asn
               180                 185                 190
   Thr Pro Ile Asn Ala Glu Lys Phe Ala Asp Pro Lys Gln Lys Ala Asp
           195                 200                 205
   Val Glu Ser Met Ile Pro Met Gly Tyr Ile Gly Glu Pro Glu Glu Ile
       210                 215                 220
   Ala Ala Val Ala Val Trp Leu Ala Ser Lys Glu Ser Ser Tyr Val Thr
   225                 230                 235                 240
   Gly Ile Thr Leu Phe Ala Asp Gly Gly Met Thr Lys Tyr Pro Ser Phe
                   245                 250                 255
   Gln Ala Gly Arg Gly
               260
<210> 2
   <211> 261
   <212> PRT
   <213> Artificial
<220>
   <223> Mutant of glucose dehydrogenase from Bacillus subtilis
<220>
   <221> MUTAGEN
   <222> (170)..(170)
<220>
   <221> MUTAGEN
   <222> (252)..(252)
<400> 2
   Met Tyr Pro Asp Leu Lys Gly Lys Val Val Ala Ile Thr Gly Ala Ala
   1               5                   10                  15
   Ser Gly Leu Gly Lys Ala Met Ala Ile Arg Phe Gly Lys Glu Gln Ala
               20                  25                  30
   Lys Val Val Ile Asn Tyr Tyr Ser Asn Lys Gln Asp Pro Asn Glu Val
           35                  40                  45
   Lys Glu Glu Val Ile Lys Ala Gly Gly Glu Ala Val Val Val Gln Gly
       50                  55                  60
   Asp Val Thr Lys Glu Glu Asp Val Lys Asn Ile Val Gln Thr Ala Ile
   65                  70                  75                  80
   Lys Glu Phe Gly Thr Leu Asp Ile Met Ile Asn Asn Ala Gly Leu Glu
                   85                  90                  95
   Asn Pro Val Pro Ser His Glu Met Pro Leu Lys Asp Trp Asp Lys Val
               100                 105                 110
   Ile Gly Thr Asn Leu Thr Gly Ala Phe Leu Gly Ser Arg Glu Ala Ile
           115                 120                 125
   Lys Tyr Phe Val Glu Asn Asp Ile Lys Gly Asn Val Ile Asn Met Ser
       130                 135                 140
   Ser Val His Glu Val Ile Pro Trp Pro Leu Phe Val His Tyr Ala Ala
   145                 150                 155                 160
   Ser Lys Gly Gly Ile Lys Leu Met Thr Lys Thr Leu Ala Leu Glu Tyr
                   165                 170                 175
   Ala Pro Lys Gly Ile Arg Val Asn Asn Ile Gly Pro Gly Ala Ile Asn
               180                 185                 190
   Thr Pro Ile Asn Ala Glu Lys Phe Ala Asp Pro Lys Gln Lys Ala Asp
           195                 200                 205
   Val Glu Ser Met Ile Pro Met Gly Tyr Ile Gly Glu Pro Glu Glu Ile
       210                 215                 220
   Ala Ala Val Ala Val Trp Leu Ala Ser Lys Glu Ser Ser Tyr Val Thr
   225                 230                 235                 240
   Gly Ile Thr Leu Phe Ala Asp Gly Gly Met Thr Leu Tyr Pro Ser Phe
                   245                 250                 255
   Gln Ala Gly Arg Gly
               260

### SEQUENCE LISTING

<110> F. Hoffmann - La Roche AG
   Roche Diagnostics GmbH
<120> Stabilisierung von Enzymen mit stabilen Coenzymen
<130> 46200P WO
<150> EP 09 168 327.6
   <151> 2009-08-20
<160> 2
<170> PatentIn version 3.3
<210> 1
   <211> 261
   <212> PRT
   <213> Artificial
<220>
   <223> Mutant of glucose dehydrogenase from Bacillus subtilis
<220>
   <221> MUTAGEN
   <222> (96)..(96)
<220>
   <221> MUTAGEN
   <222> (170)..(170)
<400> 1
   Met Tyr Pro Asp Leu Lys Gly Lys Val Val Ala Ile Thr Gly Ala Ala
   1               5                   10                  15
   Ser Gly Leu Gly Lys Ala Met Ala Ile Arg Phe Gly Lys Glu Gln Ala
               20                  25                  30
   Lys Val Val Ile Asn Tyr Tyr Ser Asn Lys Gln Asp Pro Asn Glu Val
           35                  40                  45
   Lys Glu Glu Val Ile Lys Ala Gly Gly Glu Ala Val Val Val Gln Gly
       50                  55                  60
   Asp Val Thr Lys Glu Glu Asp Val Lys Asn Ile Val Gln Thr Ala Ile
   65                  70                  75                  80
   Lys Glu Phe Gly Thr Leu Asp Ile Met Ile Asn Asn Ala Gly Leu Gly
                   85                  90                  95
   Asn Pro Val Pro Ser His Glu Met Pro Leu Lys Asp Trp Asp Lys Val
               100                 105                 110
   Ile Gly Thr Asn Leu Thr Gly Ala Phe Leu Gly Ser Arg Glu Ala Ile
           115                 120                 125
   Lys Tyr Phe Val Glu Asn Asp Ile Lys Gly Asn Val Ile Asn Met Ser
       130                 135                 140
   Ser Val His Glu Val Ile Pro Trp Pro Leu Phe Val His Tyr Ala Ala
   145                 150                 155                 160
   Ser Lys Gly Gly Ile Lys Leu Met Thr Lys Thr Leu Ala Leu Glu Tyr
                   165                 170                 175
   Ala Pro Lys Gly Ile Arg Val Asn Asn Ile Gly Pro Gly Ala Ile Asn
               180                 185                 190
   Thr Pro Ile Asn Ala Glu Lys Phe Ala Asp Pro Lys Gln Lys Ala Asp
           195                 200                 205
   Val Glu Ser Met Ile Pro Met Gly Tyr Ile Gly Glu Pro Glu Glu Ile
       210                 215                 220
   Ala Ala Val Ala Val Trp Leu Ala Ser Lys Glu Ser Ser Tyr Val Thr
   225                 230                 235                 240
   Gly Ile Thr Leu Phe Ala Asp Gly Gly Met Thr Lys Tyr Pro Ser Phe
                   245                 250                 255
   Gln Ala Gly Arg Gly
               260
<210> 2
   <211> 261
   <212> PRT
   <213> Artificial
<220>
   <223> Mutant of glucose dehydrogenase from Bacillus subtilis
<220>
   <221> MUTAGEN
   <222> (170)..(170)
<220>
   <221> MUTAGEN
   <222> (252)..(252)
<400> 2
   Met Tyr Pro Asp Leu Lys Gly Lys Val Val Ala Ile Thr Gly Ala Ala
   1               5                   10                  15
   Ser Gly Leu Gly Lys Ala Met Ala Ile Arg Phe Gly Lys Glu Gln Ala
               20                  25                  30
   Lys Val Val Ile Asn Tyr Tyr Ser Asn Lys Gln Asp Pro Asn Glu Val
           35                  40                  45
   Lys Glu Glu Val Ile Lys Ala Gly Gly Glu Ala Val Val Val Gln Gly
       50                  55                  60
   Asp Val Thr Lys Glu Glu Asp Val Lys Asn Ile Val Gln Thr Ala Ile
   65                  70                  75                  80
   Lys Glu Phe Gly Thr Leu Asp Ile Met Ile Asn Asn Ala Gly Leu Glu
                   85                  90                  95
   Asn Pro Val Pro Ser His Glu Met Pro Leu Lys Asp Trp Asp Lys Val
               100                 105                 110
   Ile Gly Thr Asn Leu Thr Gly Ala Phe Leu Gly Ser Arg Glu Ala Ile
           115                 120                 125
   Lys Tyr Phe Val Glu Asn Asp Ile Lys Gly Asn Val Ile Asn Met Ser
       130                 135                 140
   Ser Val His Glu Val Ile Pro Trp Pro Leu Phe Val His Tyr Ala Ala
   145                 150                 155                 160
   Ser Lys Gly Gly Ile Lys Leu Met Thr Lys Thr Leu Ala Leu Glu Tyr
                   165                 170                 175
   Ala Pro Lys Gly Ile Arg Val Asn Asn Ile Gly Pro Gly Ala Ile Asn
               180                 185                 190
   Thr Pro Ile Asn Ala Glu Lys Phe Ala Asp Pro Lys Gln Lys Ala Asp
           195                 200                 205
   Val Glu Ser Met Ile Pro Met Gly Tyr Ile Gly Glu Pro Glu Glu Ile
       210                 215                 220
   Ala Ala Val Ala Val Trp Leu Ala Ser Lys Glu Ser Ser Tyr Val Thr
   225                 230                 235                 240
   Gly Ile Thr Leu Phe Ala Asp Gly Gly Met Thr Leu Tyr Pro Ser Phe
                   245                 250                 255
   Gln Ala Gly Arg Gly
               260

## Beschreibung

Die vorliegende Erfindung betrifft ein Verfahren zur Stabilisierung von Enzymen durch Lagerung des Enzyms in Gegenwart eines stabilisierten Coenzyms. Weiterhin betrifft die vorliegende Erfindung ein mit einem stabilisierten Coenzym stabilisiertes Enzym sowie dessen Verwendung in Testelementen zum Nachweis von Analyten.

Diagnostische Testelemente sind wichtige Bestandteile klinisch relevanter Analyseverfahren. Hierbei steht die Messung von Analyten, z. B. Metaboliten oder Substraten, im Vordergrund, welche beispielsweise mit Hilfe eines für den Analyten spezifischen Enzyms direkt oder indirekt bestimmt werden. Die Analyten werden hierbei mit Hilfe eines Enzym-Coenzym-Komplexes umgesetzt und anschließend quantifiziert. Dabei wird der zu bestimmende Analyt mit einem geeigneten Enzym, einem Coenzym und gegenenfalls einem Mediator in Kontakt gebracht, wobei das Coenzym durch die enzymatische Reaktion physikochemisch verändert, z. B. oxidiert bzw. reduziert, wird. Sofern zusätzlich ein Mediator zum Einsatz gelangt, überträgt dieser die bei Umsetzung des Analyten freigesetzten Elektronen vom reduzierten Coenzym üblicherweise auf einen optischen Indikator oder die leitfähigen Bestandteile einer Elektrode, so dass der Vorgang beispielsweise photometrisch oder elektrochemisch erfasst werden kann. Eine Kalibrierung liefert einen direkten Zusammenhang des Messwerts mit der Konzentration des zu bestimmenden Analyten.

Ein wichtiges Kriterium bei der Bereitstellung diagnostischer Testelemente ist deren Langzeitstabilität. Aus dem Stand der Technik bekannte Testelemente, welche beispielsweise bei der Bestimmung von Blutglucose verwendet werden, weisen im Allgemeinen eine hohe Empfindlichkeit gegenüber Feuchtigkeit und Wärme auf, wobei üblicherweise speziell Coenzym und Mediator in ihrer Funktion beeinträchtigt werden. Ein weiteres Problem kommerziell erhältlicher Testelemente ist deren Empfindlichkeit gegenüber Umgebungslicht, wobei infolge Lichtabsorption durch das Enzymsystem eine Schädigung von Enzym, Coenzym oder/und Mediator auftreten kann. Bei bestimmten Anwendungsformen, z. B. bei Tests, die vom Endverbraucher selbst durchgeführt werden, können daher durch eine falsche, unbemerkte Fehllagerung des Messsystems Fehlergebnisse vorkommen, welche vom Verbraucher kaum zu erkennen sind und ggf. zu einer Fehlbehandlung der jeweiligen Erkrankung führen können.

Eine bekannte Maßnahme, die zur Erhöhung der Stabilität von diagnostischen Testelementen eingesetzt wird, ist die Verwendung stabiler Enzyme, z. B. die Verwendung von Enzymen aus thermophilen Organismen. Weiterhin besteht die Möglichkeit, Enzyme durch chemische Modifizierung, z. B. Quervernetzung, oder durch Mutagenese zu stabilisieren. Darüber hinaus können auch Enzymstabilisatoren, wie z. B. Trehalose, Polyvinylpyrrolidon und Serumalbumin, zugesetzt werden oder die Enzyme z. B. durch Photopolymerisation in Polymernetzwerke eingeschlossen werden.

Weiterhin wird versucht, die Haltbarkeit diagnostischer Testelemente durch Verwendung stabiler Mediatoren, zu verbessern. So wird durch die Verwendung von Mediatoren mit möglichst niedrigem Redoxpotential die Spezifität von Tests erhöht und Störungen während der Reaktion eliminiert. Eine untere Grenze für das Redoxpotential von Mediatoren bilden jedoch die Redoxpotentiale der Enzym/Coenzymkomplexe. Werden diese unterschritten, wird die Reaktion mit den Mediatoren verlangsamt oder gar unterbunden.

Alternativ können auch diagnostische Testelemente ohne Mediatoren verwendet werden, bei denen beispielsweise eine direkte Detektion von Coenzymen, z. B. des Coenzyms NADH, erfolgt. Ein Nachteil solcher Messsysteme besteht jedoch darin, dass native Coenzyme wie NAD und NADP instabil sind.

NAD und NADP sind basenlabile Moleküle, deren Abbauwege in der Literatur beschrieben sind (N.J. Oppenheimer, in "The Pyridine Nucleotide Coenzyms", Academic Press New York, London 1982, Hrsg. J. Everese, B. Anderson, K. You, Kapitel 3, Seiten 56-65). Im wesentlichen entsteht beim Abbau von NAD bzw. NADP ADP-Ribose, indem die Glycosyl-Bindungen zwischen der Ribose und der Pyridineinheit gespalten wird. Die reduzierten Formen NADH und NADPH sind hingegen säurelabil: z. B. ist die Epimerisierung ein bekannter Abbauweg. In beiden Fällen liegt der Instabilität von NAD/NADP und NADH/NADPH die Labilität der Glykosyl-Bindung zwischen der Ribose- und der Pyridineinheit zugrunde. Aber auch unter nicht drastischen Bedingungen, wie z. B. in wässriger Lösung, geschieht die Hydrolyse der Coenzyme NAD bzw. NADP allein schon durch die Umgebungsfeuchte. Diese Instabilität kann zu Ungenauigkeiten bei der Messung von Analyten führen.

Eine Reihe von NAD/NADP-Derivaten wird z. B. von B.M. Anderson in "The Pyridine Nucleotide Coenzymes", Academic Press New York, London 1982, Hrsg. J. Everese, B. Anderson, K. You, Kapitel 4, beschrieben. Die meisten dieser Derivate werden allerdings nicht gut von Enzymen akzeptiert. Das einzige Derivat, das daher bisher für diagnostische Tests verwendet wurde, ist 3-Acetylpyridin-Adenin-Dinukleotid (Acetyl-NAD), welches erstmals 1956 beschrieben wurde (N.O. Kaplan, J. Biol. Chem. (1956), 221, 823). Auch dieses Coenzym zeigt eine geringe Akzeptanz von Enzymen und eine Änderung im Redoxpotential.

In WO 01/94370 ist die Verwendung weiterer NAD-Derivate mit einer modifizierten Pyridin-Gruppe beschrieben. Modifikationen der Nikotinamid-Gruppe haben jedoch generell direkten Einfluss auf die katalytische Reaktion. In den meisten Fällen ist dieser Einfluss negativ.

In einem weiteren Stabilisierungskonzept wurde die Ribose-Einheit verändert, um dadurch die Stabilität der Glycosyl-Bindung zu beeinflussen. Dieses Vorgehen interferiert nicht direkt mit der katalytischen Reaktion der Nikotinamid-Gruppe. Es kann jedoch ein indirekter Einfluss bestehen, sobald das Enzym eine starke und spezifische Bindung an die Ribose-Einheit aufweist. Kaufmann et al. offenbaren diesbezüglich in WO 98/33936 und US 5,801,006 bzw. in WO 01/49247 eine Reihe von Thioribose-NAD Derivaten. Ein Zusammenhang zwischen der Modifizierung der Nikotinamid-Riboseeinheit und der Aktivität der Derivate in enzymatischen Reaktionen wurde bisher jedoch nicht gezeigt.

carbaNAD, ein Derivat ohne Glycosyl-Bindung, wurde erstmals 1988 beschrieben (J.T. Slama, Biochemistry (1988), 27, 183, und Biochemistry (1989), 28, 7688). Die Ribose wird hierin durch eine carbazyklische Zucker-Einheit substituiert. carbaNAD wurde zwar als Substrat für Dehydrogenasen beschrieben, seine Aktivität wurde bisher jedoch nicht in biochemischen Nachweisverfahren in der Klinik nachgewiesen.

Ein ähnlicher Ansatz wurde später von G.M. Blackburn (Chem. Comm. (1996), 2765) beschrieben, um carbaNAD mit einer Methylenbisphosphonat-Verbindung an Stelle des natürlichen Pyrophosphats herzustellen. Das Methylenbisphosphonat zeigt erhöhte Stabilität gegen Phosphatasen und wurde als Inhibitor für ADP-Ribosylcyclase verwendet. Eine Stabilitätserhöhung gegenüber Hydrolyse war nicht das Ziel (J.T. Slama, G.M. Blackburn).

WO 2007/012494 und US 11/460,366 offenbaren schließlich stabilisierte NAD/NADH bzw. NADP/NADPH Derivate, Enzym-Komplexe dieser Derivate und ihre Verwendung in biochemischen Nachweisverfahren und Reagenzienkits.

Osborne et al. (Biochem. J. (1976), 157, 255-259) beschreiben den Effekt verschiedener NAD-Analoga auf die Wechselwirkung zwischen einzelnen Untereinheiten von Glyceraldehyd-3-phosphat-Dehydrogenasen. Demnach bewirkt NAD⁺ durch Verstärkung der Wechselwirkung zwischen einzelnen Untereinheiten von Glyceraldehyd-3-phosphat-Dehydrogenase eine Stabilisierung des tetrameren Enzyms, während in Gegenwart von NADH eine Schwächung der intramolekularen Wechselwirkung und damit eine Destabilisierung des tetrameren Enzyms erfolgt.

EP 2 093 284 A1 offenbart ein Verfahren zur Stabilisierung eines Enzyms durch Lagerung des Enzyms in Gegenwart eines stabilen Coenzyms, ein mit einem stabilen Coenzym stabilisiertes Enzym, sowie dessen Verwendung als Nachweisreagenz zur Bestimmung eines Analyten. Als Enzym wird vorzugsweise eine Dehydrogenase ausgewählt aus Alkohol-Dehydrogenase (EC 1.1.1.1), Aminosäure-Dehydrogenase, Glucose-Dehydrogenase (EC 1.1.1.47), Glycerin-Dehydrogenase (E.C.1.1.1.6), alpha-Hydroxybutyrat-Dehydrogenase, Lactat-Dehydrogenase (EC 1.1.1.27; EC 1.1.1.28), Malat-Dehydrogenase (EC 1.1.1.37) und Sorbitol-Dehydrogenase eingesetzt.

Die der vorliegenden Erfindung zugrundeliegende Aufgabe war es, ein Verfahren zur Stabilisierung von Enzymen, insbesondere zur Langzeitstabiliserung von Enzymen, bereitzustellen, welches die oben genannten Nachteile zumindest teilweise beseitigt.

Diese Aufgabe wird erfindungsgemäß gelöst durch ein Verfahren zur Stabilisierung eines Enzyms, wobei man das Enzym in Gegenwart eines stabilisierten Coenzyms lagert. Überraschenderweise wurde gefunden, dass mit Hilfe eines stabilisierten Coenzyms eine Langzeitstabilisierung von mehreren Wochen bzw. Monaten bei hoher relativer Feuchte oder sogar in flüssiger Phase, bei erhöhten Temperaturen sowie bei Umgebungslicht möglich ist. Der Begriff "Lagerung" bedeutet in diesem Zusammenhang, dass das Enzym für eine beliebige Zeitspanne, bevorzugt über einen Zeitraum von mindestens 2 Wochen, stärker bevorzugt über einen Zeitraum von mindestens 3 Monaten, noch stärker bevorzugt über einen Zeitraum von mindestens 6 Monaten, und am stärksten bevorzugt über einen Zeitraum von mindestens 12 Monaten in Gegenwart des stabilisierten Coenzyms gehalten wird, wobei die Lagerung vorzugsweise bei Atmosphärendruck, Raumtemperatur (25°C) und einer relativen Luftfeuchtigkeit von mindestens 50% erfolgt.

Diese Erkenntnis ist überraschend, da bekannt ist, dass Enzyme in Gegenwart von nativem Coenzym zwar eine erhöhte Kurzzeitstabilität für einige Stunden besitzen (Bertoldi et al., Biochem. J. (2005), 389, 885; van den Heuvel et al., J. Biol. Chem. (2005), 280, 32115; und Pan et al., J. Chin. Biochem. Soc. (1974), 3, 1), jedoch eine geringere Haltbarkeit über einen längeren Zeitraum aufweisen (Nutrition Reviews (1978), 36, 251). Die nunmehr beobachtete Langzeitstabilität diagnostischer Testelemente, welche ein Enzym und ein stabilisiertes Coenzym umfassen, gegenüber Feuchtigkeit oder/und Wärme ist umso mehr überraschend, als stabilisierte Coenzyme eine niedrigere Bindungskonstante mit dem Enzym aufweisen als die korrespondierenden nativen Coenzyme.

Das durch das erfindungsgemäße Verfahren stabilisierte Enzym ist ein Coenzym-abhängiges Enzym ausgewählt aus der Gruppe bestehend aus einer Alkohol-Dehydrogenase (EC 1.1.1.2), einer Glucose-6-phosphat-Dehydrogenase (EC 1.1.1.49) und einer Diaphorase (EC 1.6.99.2).

Die Anmeldung offenbart des Weiteren die Verwendung einer mutierten Glucose-Dehydrogenase. Der Begriff "Mutante", wie er im Rahmen der vorliegenden Anmeldung verwendet wird, bezeichnet eine genetisch veränderte Variante eines nativen Enzyms, welche bei gleicher Anzahl an Aminosäuren eine gegenüber dem Wildtyp-Enzym veränderte Aminosäuresequenz besitzt, d.h. sich in mindestens einer Aminosäure vom Wildtyp-Enzym unterscheidet. Die Einführung der Mutation(en) kann ortsspezifisch oder nicht-ortsspezifisch, bevorzugt ortsspezifisch unter Verwendung von im Fachbereich bekannten rekombinanten Methoden, erfolgen, wobei entsprechend den jeweiligen Anforderungen und Bedingungen mindestens ein Aminosäureaustausch innerhalb der Aminosäuresequenz des nativen Enzyms resultiert. Besonders bevorzugt weist die Mutante eine gegenüber dem Wildtyp-Enzym erhöhte thermische oder hydrolytische Stabilität auf. Beispiele für derartige Mutanten werden von Baik (Appl. Environ. Microbiol. (2005), 71, 3285), Vásquez-Figueroa (ChemBioChem (2007), 8, 2295) sowie in WO 2005/045016 A2 beschrieben, auf deren Offenbarung hiermit ausdrücklich Bezug genommen wird.

Die mutierte Glucose-Dehydrogenase kann die gegenüber der korrespondierenden Wildtyp-Glucose-Dehydrogenase veränderte(n) Aminosäure(n) grundsätzlich an einer beliebigen Position ihrer Aminosäuresequenz enthalten. Vorzugsweise umfasst die mutierte Glucose-Dehydrogenase eine Mutation an mindestens einer der Positionen 96, 170 und 252 der Aminosäuresequenz der Wildtyp-Glucose-Dehydrogenase, wobei Mutanten mit Mutationen an Position 96 und Position 170 bzw. Mutationen an Position 170 und Position 252 besonders bevorzugt sind. Als vorteilhaft hat es sich erwiesen, wenn die mutierte Glucose-Dehydrogenase neben diesen Mutationen keine weiteren Mutationen enthält.

Die Mutation an den Positionen 96, 170 und 252 kann grundsätzlich einen beliebigen Aminosäureaustausch umfassen, welcher zu einer Stabilisierung, z. B. einer Erhöhung der thermischen oder hydrolytischen Stabilität, des Wildtyp-Enzyms führt. Vorzugsweise umfasst die Mutation an Position 96 einen Aminosäureaustausch von Glutaminsäure gegen Glycin, während in Bezug auf Position 170 ein Aminosäureaustausch von Glutaminsäure gegen Arginin oder Lysin, insbesondere ein Aminosäureaustausch von Glutaminsäure gegen Lysin, bevorzugt ist. Was die Mutation an Position 252 anbetrifft, so umfasst diese bevorzugt einen Aminosäureaustausch von Lysin gegen Leucin.

Die mutierte Glucose-Dehydrogenase kann durch Mutation einer aus einer beliebigen biologischen Quelle stammenden Wildtyp-Glucose-Dehydrogenase erhalten werden, wobei der Begriff "biologische Quelle" im Sinne dieser Erfindung sowohl Prokaryoten, wie beispielsweise Bakterien, als auch Eukaryoten, wie beispielsweise Säuger und andere Tiere, umfasst. Vorzugsweise entstammt die Wildtyp-Glucose-Dehydrogenase einem Bakterium, wobei es sich besonders bevorzugt um eine Glucose-Dehydrogenase aus Bacillus megaterium, Bacillus subtilis oder Bacillus thuringiensis, insbesondere aus Bacillus subtilis, handelt.

Besonders bevorzugt handelt es sich bei der mutierten Glucose-Dehydrogenase um eine durch Mutation von Wildtyp-Glucose-Dehydrogenase aus Bacillus subtilis erhaltene Glucose-Dehydrogenase, welche die in SEQ ID NO: 1 (GlucDH_E96G_E170K) oder die in SEQ ID NO: 2 (GlucDH_E170K_K252L) dargestellte Aminosäuresequenz besitzt.

Ein stabilisiertes Coenzym im Sinne der vorliegenden Erfindung ist ein gegenüber dem nativen Coenzym chemisch verändertes Coenzym, welches bei Atmosphärendruck eine im Vergleich zum nativen Coenzym höhere Stabilität gegenüber Feuchtigkeit, Temperaturen insbesondere im Bereich von 0°C bis 50°C, Säuren und Basen insbesondere im Bereich von pH 4 bis pH 10, oder/und Nukleophilen wie beispielsweise Alkoholen oder Aminen, aufweist und insofern unter identischen Umgebungsbedingungen über einen längeren Zeitraum als das native Coenzym seine Wirkung entfalten kann. Vorzugsweise weist das stabilisierte Coenzym eine im Vergleich zum nativen Coenzym höhere hydrolytische Stabilität auf, wobei eine vollständige Hydrolysestabilität unter Testbedingungen besonders bevorzugt ist. Im Vergleich zum nativen Coenzym kann das stabilisierte Coenzym eine verringerte Bindungskonstante für das Enzym aufweisen, beispielsweise eine um den Faktor von 2 oder mehr verringerte Bindungskonstante.

Bevorzugte Beispiele für stabilisierte Coenzyme sind stabilisierte Derivate von Nikotinamid-Adenin-Dinukleotid (NAD/NADH) oder Nikotinamid-Adenin-Dinukleotidphosphat (NADP/NADPH), oder verkürzte NAD-Derivate, z. B. ohne AMP-Teil bzw. mit nicht-nukleosidischen Resten, z. B. hydrophoben Resten. Ebenfalls bevorzugt ist als stabilisiertes Coenzym im Sinne der vorliegenden Erfindung ist die Verbindung der Formel (I)

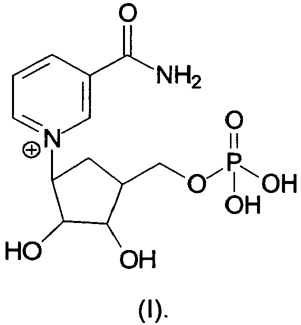

Bevorzugte stabilisierte Derivate von NAD/NADH bzw. NADP/NADPH sind in den zuvor genannten Referenzen beschrieben, auf deren Offenbarung hiermit ausdrücklich Bezug genommen wird. Besonders bevorzugte stabilisierte Coenzyme sind in WO 2007/012494 bzw. US 11/460,366 beschrieben, auf deren Offenbarung hiermit ausdrücklich Bezug genommen wird. Das stabilisierte Coenzym wird besonders bevorzugt aus Verbindungen der allgemeinen Formel (II) ausgewählt: 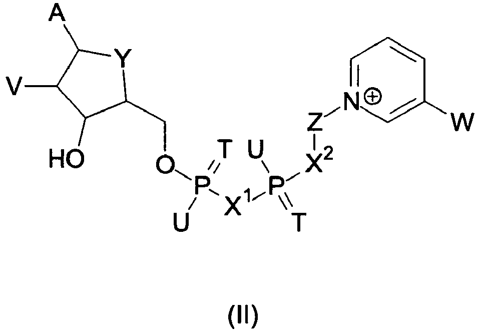 mit
- A =: Adenin oder ein Analog davon,
- T =: jeweils unabhängig O, S,
- U =: jeweils unabhängig OH, SH, BH₃⁻, BCNH₂⁻,
- V =: jeweils unabhängig OH oder eine Phosphatgruppe, oder zwei Gruppen, die eine cyclische Phosphatgruppe bilden;
- W =: COOR, CON(R)₂, COR, CSN(R)₂ mit R = jeweils unabhängig H oder C₁-C₂-Alkyl,
- X¹, X² =: jeweils unabhängig O, CH₂, CHCH₃, C(CH₃)₂, NH, NCH₃,
- Y =: NH, S, O, CH₂,
- Z =: ein linearer oder cyclischer organischer Rest ist,
mit der Maßgabe, dass Z und der Pyridin-Rest nicht durch eine glycosidische Verbindung verknüpft sind, oder ein Salz oder gegebenenfalls eine reduzierte Form davon.

In den Verbindungen der Formel (II) ist Z vorzugsweise ein linearer Rest mit 4-6 C-Atomen, vorzugsweise 4 C-Atomen, worin 1 oder 2 C-Atome gegebenenfalls durch ein oder mehrere Heteroatome ausgewählt aus O, S und N ersetzt sind, oder ein Rest umfassend eine cyclische Gruppe mit 5 oder 6 C-Atomen, die gegebenenfalls ein Heteroatom ausgewählt aus O, S und N sowie gegebenenfalls einen oder mehrere Substituenten enthält, und einen Rest CR⁴₂, wobei CR⁴₂ an die cyclische Gruppe und an X² gebunden ist, mit R⁴ = jeweils unabhängig H, F, Cl, CH₃.

Besonders bevorzugt ist Z ein gesättiger oder ungesättigter carbocyclischer oder heterocyclischer Fünfring, insbesondere eine Verbindung der allgemeinen Formel (III) 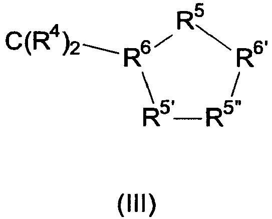 wobei zwischen R⁵' und R⁵" eine Einfach- oder Doppelbindung vorliegen kann, mit
- R⁴ =: jeweils unabhängig H, F, Cl, CH₃,
- R⁵ =: CR⁴₂,
- R⁵' =: O, S, NH, NC₁-C₂-Alkyl, CR⁴₂, CHOH, CHOCH₃, und R⁵" = CR⁴₂, CHOH, CHOCH₃, wenn zwischen R⁵' und R⁵" eine Einfachbindung vorliegt,
- R⁵' = R⁵" = CR⁴,: wenn zwischen R⁵' und R⁵" eine Doppelbindung vorliegt, und
- R⁶, R^{6'} =: jeweils unabhängig CH oder CCH₃.

In einer bevorzugten Ausführungsform enthalten die Verbindungen der Formel (II) Adenin oder Adenin-Analoga, wie z. B. C₈- und N₆-substituiertes Adenin, Deaza-Varianten wie 7-Deaza, Azavarianten wie 8-Aza oder Kombinationen wie 7-Deaza oder 8-Aza oder carbocyclische Analoga, wie Formycin, wobei die 7-Deazavarianten in der 7-Position mit Halogen, C₁-C₆-Alkinyl, -Alkenyl oder -Alkyl substituiert sein können.

In einer weiteren bevorzugten Ausführungsform enthalten die Verbindungen der Formel (II) Adenosin-Analoga, welche an Stelle von Ribose z. B. 2-Methoxy-deoxyribose, 2'-Fluoro-deoxyribose, Hexitol, Altritol bzw. polycyclische Analoga, wie Bicyclo-, LNA- und Tricyclo-Zucker, enthalten.

Insbesondere können in den Verbindungen der Formel (II) auch (Di-)-Phosphatsauerstoffe isotronisch ersetzt sein, wie z. B. O⁻ durch S⁻ bzw. BH₃⁻, O durch NH, NCH₃ bzw. CH₂ und =O durch =S. In den Verbindungen der Formel (II) ist W vorzugsweise CONH₂ oder COCH₃.

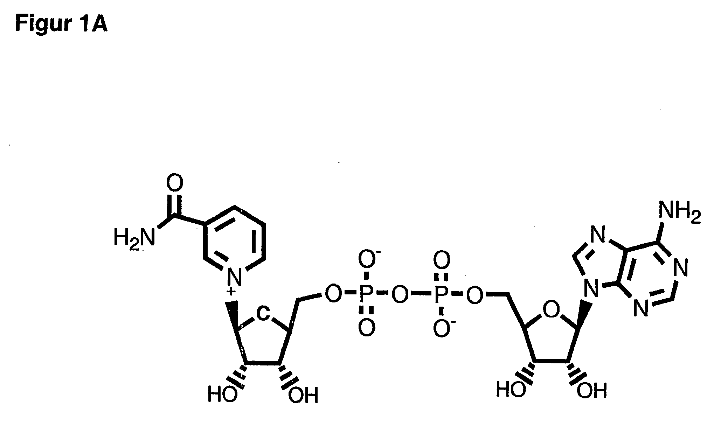

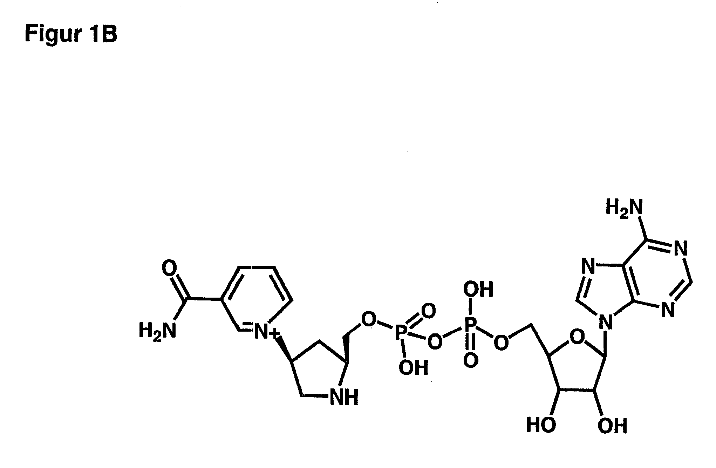

In den Gruppen der Formel (III) ist R⁵ vorzugsweise CH₂. Weiterhin ist bevorzugt, dass R⁵' ausgewählt ist aus CH₂, CHOH und NH. In einer besonders bevorzugten Ausführungsform sind R⁵' und R⁵" jeweils CHOH. In noch einer weiteren bevorzugten Ausführungsform ist R⁵' NH und R⁵" CH₂. Stärker bevorzugt ist eine Verbindung der Formel (III), in welcher R⁴ = H ist, R⁵ = CH₂ ist, R⁵' = R⁵" = CHOH ist, und R⁶ = R⁶' = CH ist. Spezifische Beispiele für bevorzugte stabilisierte Coenzyme sind in Figur 1A und 1B abgebildet. In der am stärksten bevorzugten Ausführungsform handelt es sich bei dem stabilisierten Coenzym um carbaNAD.

Das erfindungsgemäße Verfahren ist insbesondere zur Langzeitstabilisierung von Enzymen geeignet. Dies bedeutet, dass man das stabilisierte Enzym, z. B. als Trockensubstanz oder in flüssiger Phase, beispielsweise über eine Dauer von mindestens 2 Wochen, bevorzugt von mindestens 4 Wochen, und am stärksten bevorzugt von mindestens 8 Wochen lagert, wobei die Enzymaktivität vorzugsweise um weniger als 50%, stärker bevorzugt um weniger als 30%, und am stärksten bevorzugt um weniger als 20%, bezogen auf den Ausgangswert der Enzymaktivität, abnimmt.

Weiterhin umfasst das erfindungsgemäße Verfahren eine Lagerung des stabilisierten Enzyms bei erhöhten Temperaturen, beispielsweise bei einer Temperatur von mindestens 20°C, bevorzugt von mindestens 25°C, und am stärksten bevorzugt von mindestens 30°C, wobei die Enzymaktivität dabei bevorzugt um weniger als 50%, stärker bevorzugt um weniger als 30%, und am stärksten bevorzugt um weniger als 20%, bezogen auf den Ausgangswert der Enzymaktivität, abnimmt. Die Lagerung kann hierbei gegebenenfalls für eine längere Zeit, wie oben angegeben, durchgeführt werden.

Überdies sieht das erfindungsgemäße Verfahren eine Lagerung des stabilisierten Enzyms in Gegenwart von Umgebungslicht, d. h. in Gegenwart von Licht mit einer Wellenlänge von ≥ 300 nm, vor, wobei die Enzymaktivität bevorzugt um weniger als 50%, stärker bevorzugt um weniger als 30%, und am stärksten bevorzugt um weniger als 20%, bezogen auf den Ausgangswert der Enzymaktivität, abnimmt. Die Lagerung kann hierbei gegebenenfalls für eine längere Zeit oder/und bei erhöhten Temperaturen, wie oben jeweils angegeben, durchgeführt werden. Aufgrund der Stabilität des Enzymsystems gegenüber Umgebungslicht kann das stabilisierte Enzym zudem kurz vor Benutzung oder/und nach Entnahme aus einer Verpackung auch direktem Sonnenlicht ausgesetzt werden.

Durch die erfindungsgemäße Stabilisierung ist es möglich, das stabilisierte Enzym auch ohne Trocknungsmittel oder/und bei einer hohen relativen Luftfeuchtigkeit, z. B. einer relativen Luftfeuchtigkeit von mindestens 50%, zu lagern, wobei die Enzymaktivität bevorzugt um weniger als 50%, stärker bevorzugt um weniger als 30%, und am stärksten bevorzugt um weniger als 20%, bezogen auf den Ausgangswert der Enzymaktivität, abnimmt. Die Lagerung kann hierbei gegebenenfalls für eine längere Zeit, bei erhöhten Temperaturen oder/und in Gegenwart von Umgebungslicht, wie oben jeweils angegeben, durchgeführt werden. Verfahren bzw. Tests zur Bestimmung der Aktivität von Enzymen sind im Stand der Technik weithin bekannt und können vom Fachmann, sofern erforderlich, den jeweiligen Bedürfnissen angepasst werden, wobei zum Vergleich der Enzymaktivität vor und nach der Lagerung jeweils die gleichen Testbedingungen verwendet werden.

Die Lagerung des stabilisierten Enzyms kann einerseits als Trockensubstanz und andererseits in Flüssigphase erfolgen. Bevorzugt erfolgt die Lagerung des stabilisierten Enzyms auf oder in einem Testelement, das zur Bestimmung eines Analyten geeignet ist. Das stabilisierte Enzym ist dabei vorzugsweise Bestandteil eines Nachweisreagenzes, welches gegebenenfalls noch weitere Bestandteile wie etwa Mediatoren, optische Indikatoren, Salze, Puffer, etc. enthalten kann.

Das stabilisierte Enzym kann zum Nachweis von Analyten, beispielsweise von Parametern in Körperflüssigkeiten wie etwa Blut, Serum, Plasma oder Urin bzw. in Abwasserproben oder Lebensmitteln, eingesetzt werden. Als Analyten können beliebige biologische oder chemische Substanzen bestimmt werden, die durch eine Redoxreaktion nachgewiesen werden können, z. B. Substanzen, bei denen es sich um Substrate eines Coenzymabhängigen Enzyms handelt oder Coenzym-abhängige Enzyme selbst. Bevorzugte Beispiele für Analyten sind Glucose, Milchsäure, Äpfelsäure, Glycerin, Alkohol, Cholesterin, Triglyceride, Ascorbinsäure, Cystein, Glutathion, Peptide, Harnstoff, Ammonium, Salicylat, Pyruvat, 5'-Nukleotidase, Creatinkinase (CK), Lactat-Dehydrogenase (LDH), Kohlendioxid etc. Bevorzugt ist der Analyt Glucose.

Ein weiterer Gegenstand der vorliegenden Erfindung ist die Verwendung einer erfindungsgemäßen Verbindung oder eines erfindungsgemäß stabilisierten Enzyms zum Nachweis eines Analyten in einer Probe durch eine enzymatische Reaktion. Besonders bevorzugt ist hierbei der Nachweis von Glucose mit Hilfe einer Glucose-Dehydrogenase (EC 1.1.1.47) oder einer Glucose-6-phosphat-Dehydrogenase (EC 1.1.1.49) unter Verwendung geeigneter Coenzyme.

Die Veränderung des stabilisierten Coenzyms durch Reaktion mit dem Analyten kann grundsätzlich auf beliebige Art und Weise nachgewiesen werden. Hier können grundsätzlich alle aus dem Stand der Technik bekannten Methoden zum Nachweis enzymatischer Reaktionen eingesetzt werden. Vorzugsweise wird jedoch die Veränderung des Coenzyms durch optische Methoden nachgewiesen. Optische Nachweismethoden umfassen beispielsweise die Messung von Absorption, Fluoreszenz, Circulardichroismus (CD), optische Rotationsdispersion (ORD), Refraktometrie etc.

Optische Nachweisverfahren, welche im Rahmen der vorliegenden Anmeldung bevorzugt Anwendung finden, sind Photometrie und Fluorimetrie. Zur photometrischen Messung einer Veränderung des Coenzyms infolge Umsetzung mit dem Analyten bedarf es indessen der zusätzlichen Anwesenheit mindestens eines Mediators, welcher die Reaktivität des reduzierten Coenzyms erhöht und eine Übertragung von Elektronen auf einen geeigneten optischen Indikator oder ein optisches Indikatorsystem ermöglicht.

Als Mediatoren, welche für die Zwecke der vorliegenden Erfindung geeignet sind, kommen u.a. Nitrosoaniline, wie beispielsweise [(4-Nitrosophenyl)imino]dimethanol-Hydrochlorid, Chinone, wie beispielsweise Phenanthrenchinone, Phenanthrolinchinone oder Benzo[h]-chinolinchinone, Phenazine, wie beispielsweise 1-(3-Carboxypropoxy)-5-ethylphenazinium-trifluormethansulfonat, oder/und Diaphorase (EC 1.6.99.2) in Frage.

Diaphorasen besitzen insbesondere gegenüber Phenazinen den Vorteil einer höheren Stabilität, können jedoch durch Abbauprodukte von nativen Coenzymen, z. B. durch Abbauprodukte von NAD oder NADP, in ihrer Funktion beeinträchtigt werden, wie beispielsweise aus DE 2 061 984 A bekannt ist.

Bevorzugte Beispiele für Phenanthrolinchinone im Sinne der vorliegenden Erfindung umfassen 1,10-Phenanthrolin-5,6-chinone, 1,7-Phenanthrolin-5,6-chinone, 4,7-Phenanthrolin-5,6-chinone sowie deren N-alkylierte oder N,N'-dialkylierte Salze, wobei im Falle N-alkylierter bzw. N,N'-dialkylierter Salze Halogenide, Trifluormethansulfonat oder andere die Löslichkeit erhöhende Anionen als Gegenion bevorzugt sind. Diaphorasen, welche für die Zwecke der vorliegenden Erfindung besonders geeignet sind, umfassen beispielsweise Diaphorase aus Schweineherz, Clostridium kluyverii und Bacillus stearothermophilus sowie die in US 2007/0196899 A1 beschriebene Diaphorase-Mutante, welche eine gegenüber nativen Diaphorasen verbesserte katalytische Funktion und Thermostabilität aufweist. Auf die Offenbarung obiger US-Anmeldung wird hiermit ausdrücklich Bezug genommen.

Als optischer Indikator oder als optisches Indikatorsystem kann eine beliebige Substanz verwendet werden, welche reduzierbar ist und bei Reduktion eine detektierbare Änderung ihrer optischen Eigenschaften, wie beispielsweise Farbe, Fluoreszenz, Remission, Transmission, Polarisation oder/und Brechungsindex, erfährt. Die Bestimmung des Vorhandenseins oder/und der Menge des Analyten in der Probe kann mit dem bloßen Auge oder/und mittels einer Detektionsvorrichtung unter Verwendung eines dem Fachmann geeignet erscheinenden photometrischen Verfahrens erfolgen. Vorzugsweise werden Heteropolysäuren, und insbesondere 2,18-Phosphormolybdänsäure, als optische Indikatoren verwendet, die zum entsprechenden Heteropolyblau reduziert werden. Alternativ können auch Chinone wie beispielsweise Resazurin, Dichlorphenolindophenol oder/und Tetrazoliumsalze als optische Indikatoren eingesetzt werden. Tetrazoliumsalze, welche für die Zwecke der vorliegenden Erfindung besonders geeignet sind, umfassen beispielsweise die kommerziell erhältlichen Produkte WST-3, WST-4 und WST-5 (alle Fa. Dojindo), sind jedoch nicht auf diese beschränkt.

Besonders bevorzugt wird die Veränderung des Coenzyms durch Messung der Fluoreszenz nachgewiesen. Die Fluoreszenzmessung ist hochsensitiv und ermöglicht den Nachweis selbst geringer Konzentrationen des Analyten in miniaturisierten Systemen. Alternativ kann die Veränderung des Coenzyms auch elektrochemisch unter Verwendung eines geeigneten Testelements, wie beipielsweise eines elektrochemischen Teststreifens, nachgewiesen werden. Voraussetzung hierfür ist wiederum die Verwendung geeigneter Mediatoren, welche vom reduzierten Coenzym durch Übertragung von Elektronen in eine reduzierte Form überführt werden können. Die Bestimmung des Analyten erfolgt über eine Messung des zur Reoxidation des reduzierten Mediators benötigten Stromes, welcher mit der Konzentration des Analyten in der Probe korreliert. Beispiele für Mediatoren, welche für elektrochemische Messungen verwendet werden können, umfassen insbesondere die vorstehend erwähnten, für photometrische Messungen eingesetzten Mediatoren.

Zum Nachweis eines Analyten kann ein Flüssigtest verwendet werden, wobei das Reagenz z. B. in Form einer Lösung oder Suspension in einer wässrigen oder nichtwässrigen Flüssigkeit, oder als Pulver oder Lyophilisat vorliegt. Es kann jedoch auch ein Trockentest verwendet werden, wobei das Reagenz auf einem Träger aufgebracht ist. Der Träger kann beispielsweise einen Teststreifen, umfassend ein saugfähiges oder/und quellbares Material, umfassen, das von der zu untersuchenden Probeflüssigkeit benetzt wird.

Ein besonders bevorzugtes Testformat umfasst die Verwendung des Enzyms Glucose-6-phosphat-Dehydrogenase mit einem stabilisierten NAD-Derivat zum Nachweis von Glucose insbesondere in einem Nasstest, wobei ein Derivat des reduzierten Coenzyms NADH gebildet wird. Der Nachweis von NADH erfolgt durch optische Methoden, z. B. durch photometrische oder fluorometrische Bestimmung nach UV-Anregung. Ein besonders bevorzugtes Testsystem ist in US 2005/0214891 beschrieben, auf das hier ausdrücklich Bezug genommen wird.

In einem weiteren Aspekt betrifft die Erfindung ein mit einem stabilisierten Coenzym stabilisiertes Enzym, welches bei einer Lagerung von mindestens 2 Wochen, bevorzugt von mindestens 4 Wochen, und am stärksten bevorzugt von mindestens 8 Wochen bei einer Temperatur von mindestens 20°C, bevorzugt von mindestens 25°C, und am stärksten bevorzugt von mindestens 30°C oder/und in Gegenwart von Licht mit einer Wellenlänge von ≥ 300 nm, gegebenenfalls bei hoher Luftfeuchtigkeit oder/und in Abwesenheit von Trocknungsmittel, eine Abnahme der Enzymaktivität von weniger als 50%, bevorzugt von weniger als 30%, und am stärksten bevorzugt von weniger als 20%, bezogen auf den Ausgangswert der Enzymaktivität, aufweist. Hierbei gelangt ein Enzym zum Einsatz, wie es zuvor angegeben ist.

In noch einem weiteren Aspekt betrifft die Erfindung ein Nachweisreagenz zur Bestimmung eines Analyten, welches ein stabilisiertes Enzym, wie zuvor angegeben, enthält. Außerdem betrifft die Erfindung ein Testelement, welches ein erfindungsgemäß stabilisiertes Enzym bzw. ein erfindungsgemäßes Nachweisreagenz enthält. Das Nachweisreagenz bzw. das Testelement können zur Durchführung von Trocken- oder Flüssigtests geeignet sein. Bevorzugt ist das Testelement ein Teststreifen zum fluorometrischen oder photometrischen Nachweis eines Analyten. Ein solcher Teststreifen enthält das stabilisierte Enzym immobilisiert auf einem saugfähigen oder/und quellbaren Material, wie etwa Cellulose, Kunststoff, etc.

Die Anmeldung offenbart weiterhin ein Verfahren zur Stabilisierung eines Enzyms insbesondere gegenüber Umgebungslicht, wobei das Enzym, wie es zuvor angegeben ist, in Gegenwart eines nativen

Coenzyms gelagert wird. In einer bevorzugten Variante gelangt als Enzym eine Alkohol-Dehydrogenase (EC 1.1.1.1; EC 1.1.1.2), eine Glucose-Dehydrogenase (EC 1.1.1.47), eine Glucose-6-phosphat-Dehydrogenase (EC 1.1.1.49) oder eine Diaphorase (EC 1.6.99.2) zum Einsatz, wobei Glucose-Dehydrogenasen (EC 1.1.1.47), umfassend native und mutierte Glucose-Dehydrogenasen, und Glucose-6-phosphat-Dehydrogenasen (EC 1.1.1.49) als besonders bevorzugt gelten. Als natives Coenzym dient bevorzugt eine native Nikotinamid-Adenin-Dinukleotid-(NAD/NADH)- oder eine native Nikotinamid-Adenin-Dinukleotidphosphat-(NADP/NADPH)-Verbindung, und insbesondere natives NAD bzw. NADP.

Die Erfindung soll durch die nachfolgenden Figuren und Beispiele näher erläutert werden:

### Beschreibung der Figuren

**Figur 1A****:** Darstellung des stabilisierten Coenzyms carbaNAD (cNAD).
**Figur 1B****:** Darstellung des stabilisierten Coenzyms Pyrrolidinyl-NAD.
**Figur 2****:** Darstellung der Ergebnisse der Enzymkinetik von Glucose-Dehydrogenase in Gegenwart von NAD bzw. von Glucose-Dehydrogenase in Gegenwart von cNAD vor und nach Lagerung.
   **2A:** Kinetik von GlucDH in Gegenwart von NAD nach 1 Tag.
   **2B:** Kinetik von GlucDH in Gegenwart von cNAD nach 1 Tag.
   **2C:** Kinetik von GlucDH in Gegenwart von NAD nach 5 Wochen Lagerung bei 32 °C und 85 % relativer Luftfeuchtigkeit.
   **2D:** Kinetik von GlucDH in Gegenwart von cNAD nach 5 Wochen Lagerung bei 32 °C und 85 % relativer Luftfeuchtigkeit.

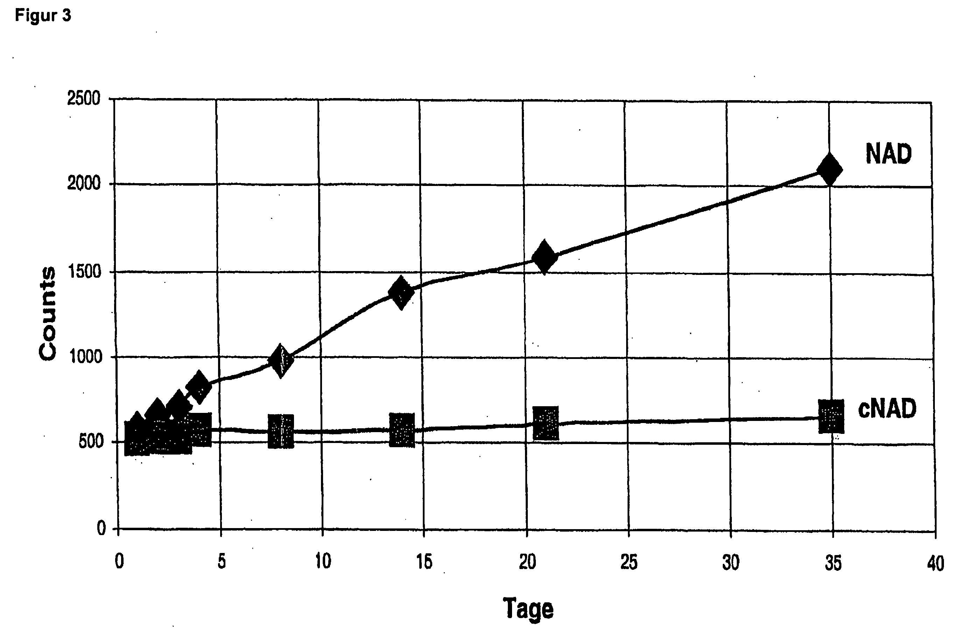

**Figur 3****:** Vergleich der Blankwerte von Glucose-Dehydrogenase in Gegenwart von NAD bzw. von GlucDH in Gegenwart von cNAD über einen Zeitraum von bis zu 5 Wochen bei 32 °C und 85 % Luftfeuchtigkeit.

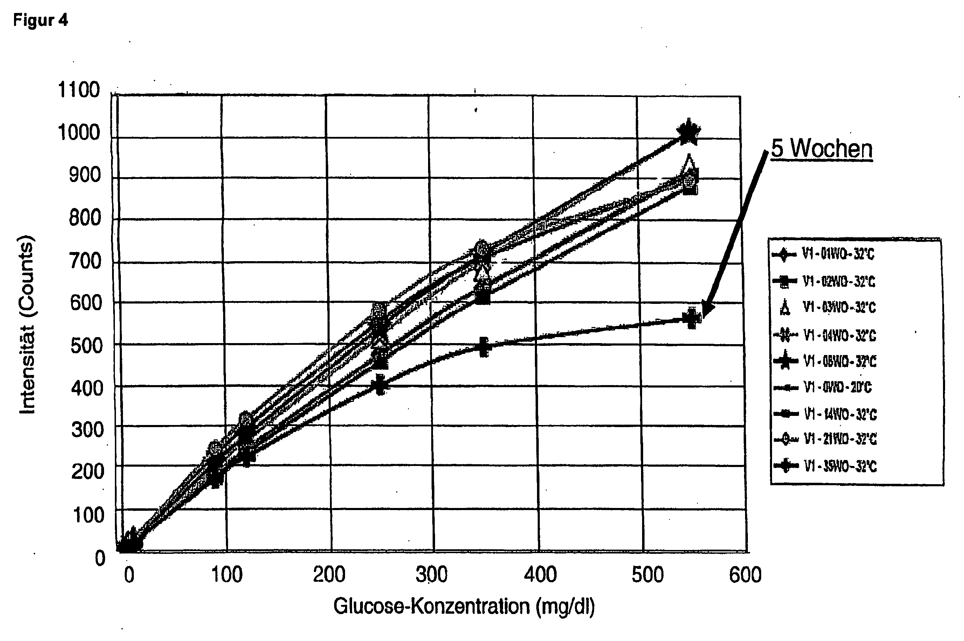

**Figur 4****:** Darstellung verschiedener Funktionskurven von Glucose-Dehydrogenase nach Lagerung von Glucose-Dehydrogenase in Gegenwart von NAD bei 32 °C und 85 % Luftfeuchtigkeit. Die Lagerungsdauer variierte zwischen 1 Tag und 5 Wochen.

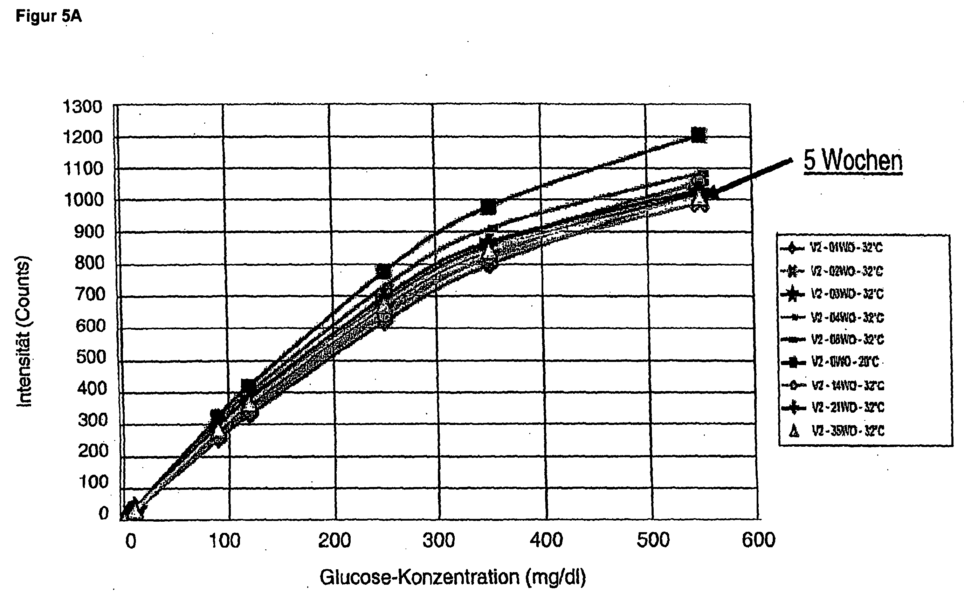

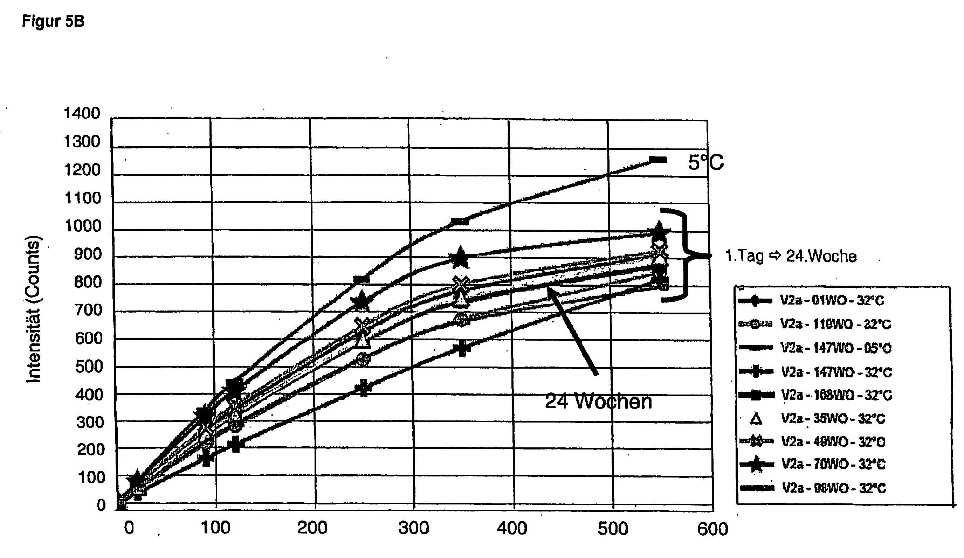

**Figur 5****:** Darstellung verschiedener Funktionskurven von Glucose-Dehydrogenase nach Lagerung von Glucose-Dehydrogenase in Gegenwart von cNAD bei 32 °C und 85 % Luftfeuchtigkeit. Die Lagerungsdauer variierte zwischen 1 Tag und 5 Wochen (Figur 5A) bzw. zwischen 1 Tag und 24 Wochen (Figur 5B).

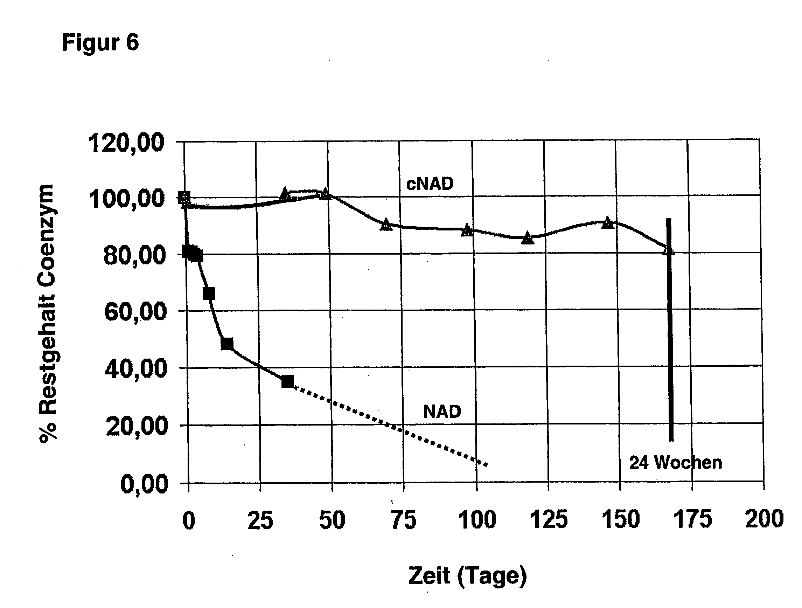

**Figur 6****:** Darstellung des Restgehaltes an NAD bzw. cNAD nach Lagerung von Glucose-Dehydrogenase in Gegenwart von NAD bzw. cNAD über 24 Wochen bei 32 °C und 85 % Luftfeuchtigkeit.

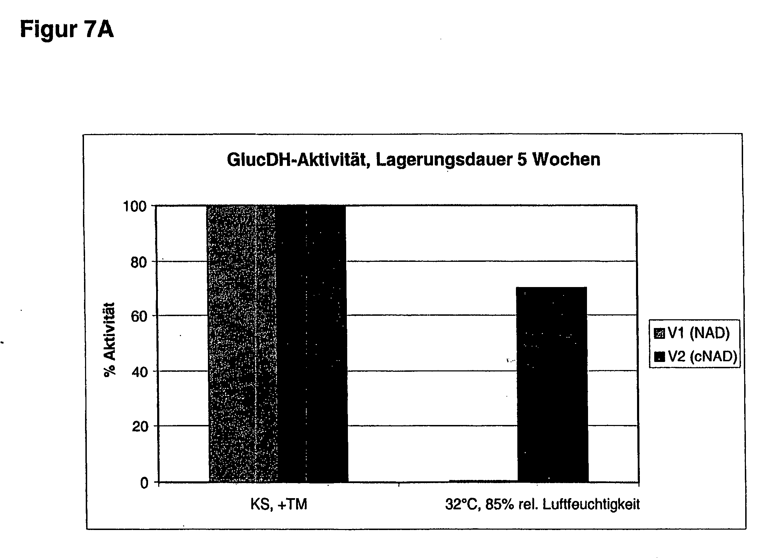

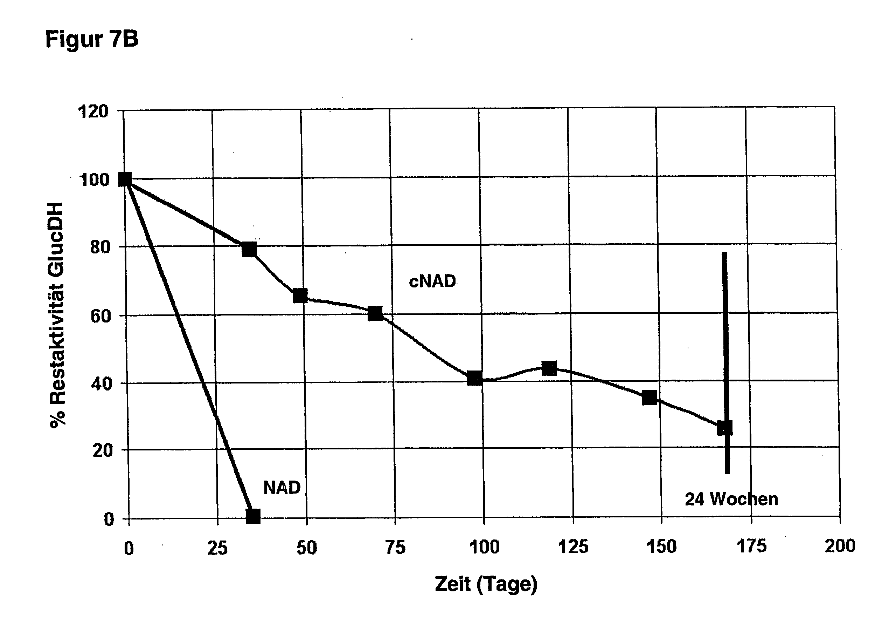

**Figur 7****:** Darstellung der GlucDH-Aktivität nach Lagerung von Glucose-Dehydrogenase in Gegenwart von NAD bzw. cNAD über 5 Wochen (Figur 7A) bzw. 24 Wochen (Figur 7B) bei 32 °C und 85 % Luftfeuchtigkeit.

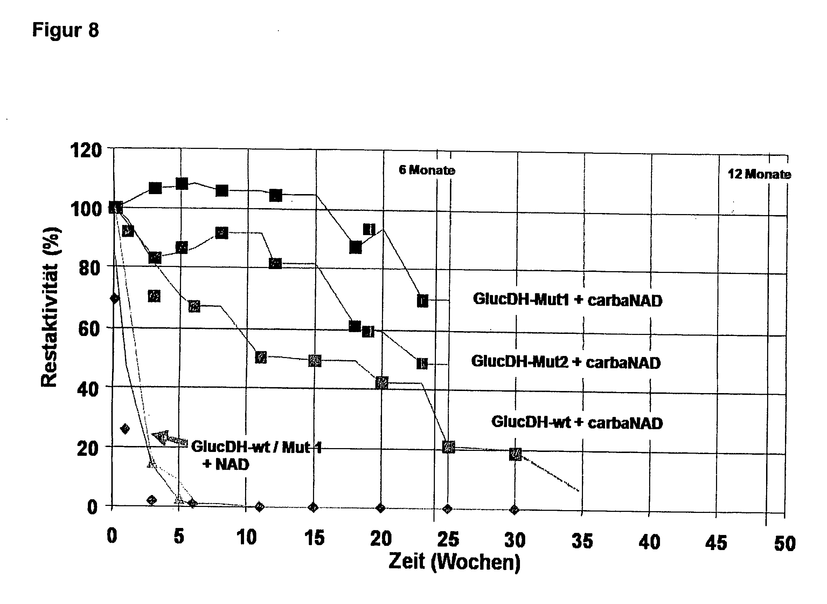

**Figur 8****:** Darstellung der GlucDH-Aktivität nach Lagerung von Glucose-Dehydrogenase (GlucDH-wt), der Doppelmutante GlucDH_E96G_E170K (GlucDH-Mut1) und der Doppelmutante GlucDH_E170K_K252L (GlucDH-Mut2) über einen Zeitraum von 25 Wochen in Gegenwart von NAD bzw. cNAD bei 32°C und 83% relativer Luftfeuchtigkeit.

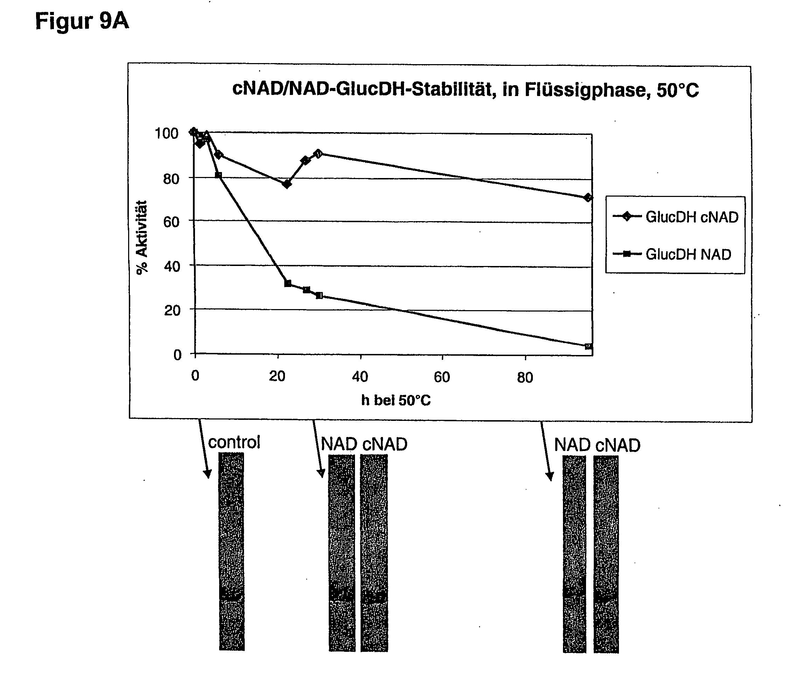

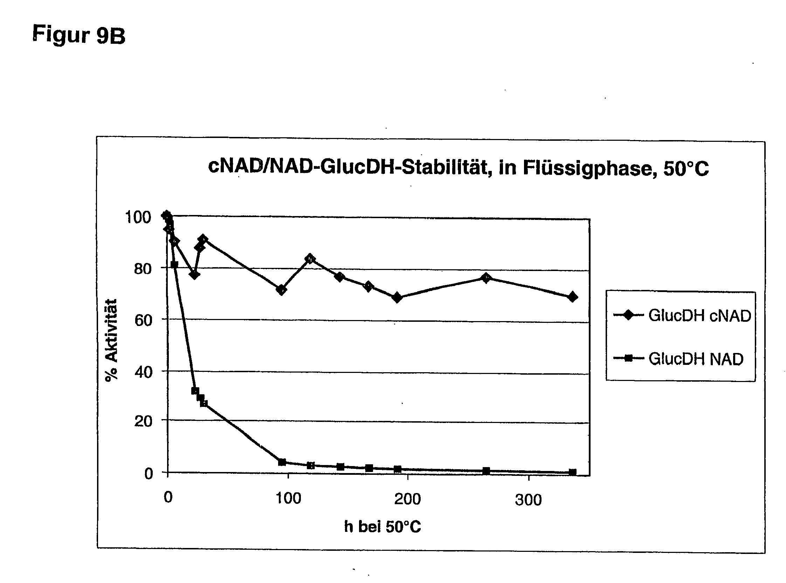

**Figur 9****:** Darstellung der Stabilität von Glucose-Dehydrogenase in Gegenwart von NAD bzw. cNAD in Flüssigphase bei 50 °C über einen Zeitraum von 4 Tagen (Figur 9A) bzw. 14 Tagen (Figur 9B). Testbedingungen: GlucDH 10 mg/ml; NAD bzw. cNAD 12 mg/ml; Puffer: 0,1 M Tris, 1,2 M NaCl, pH 8,5; Temperatur 50 °C.

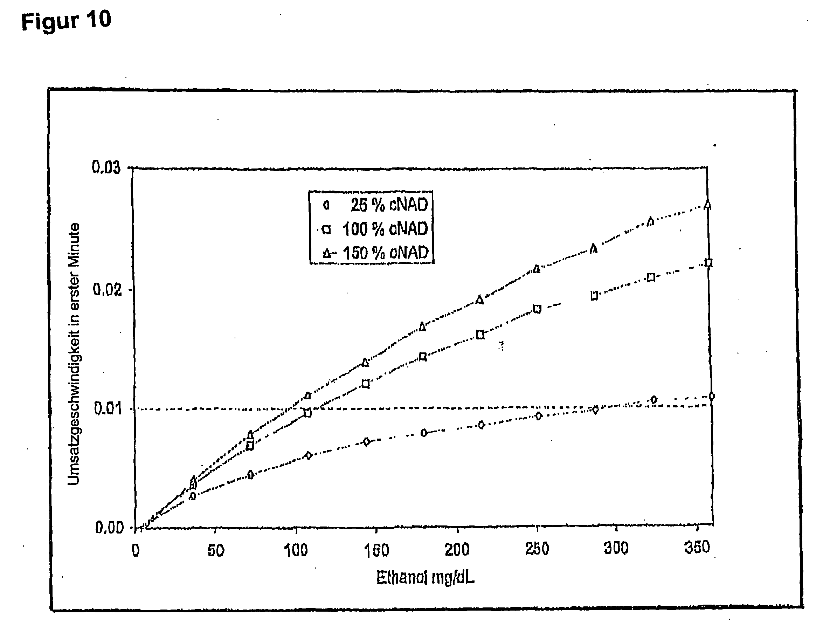

**Figur 10****:** Darstellung verschiedener Funktionskurven von Alkohol-Dehydrogenase in Gegenwart von cNAD. Die Konzentration an cNAD variierte zwischen 25% und 150%, bezogen auf die ursprüngliche Konzentration an NAD im Flüssigtest.

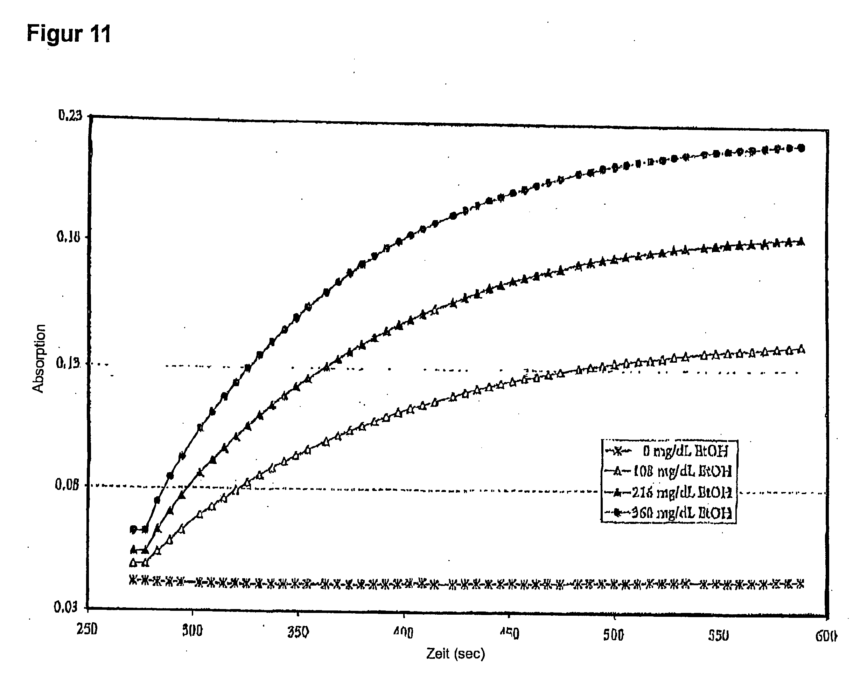

**Figur 11****:** Darstellung der Ergebnisse der Enzymkinetik von Alkohol-Dehydrogenase in Gegenwart von cNAD bei verschiedenen Ethanol-Konzentrationen.

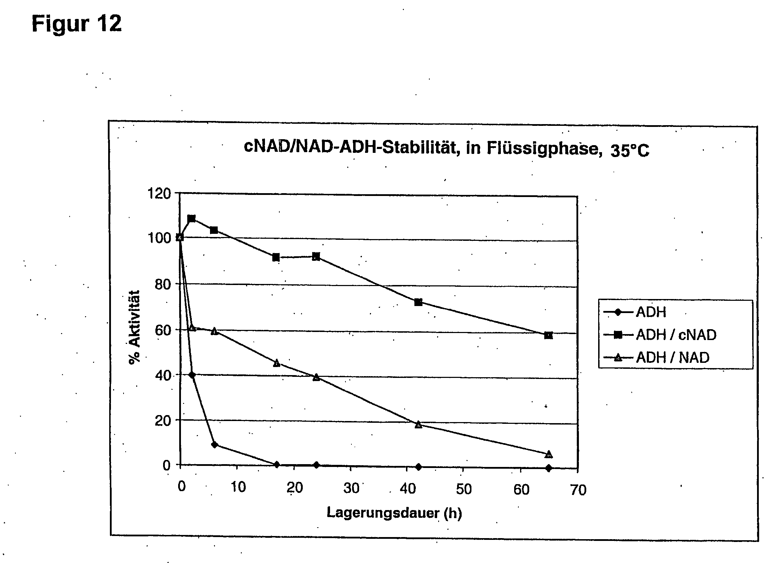

**Figur 12****:** Darstellung der Stabilität von Alkohol-Dehydrogenase aus Hefe in Gegenwart von NAD bzw. cNAD in Flüssigphase bei 35 °C über einen Zeitraum von 65 Stunden. Testbedingungen: ADH 5 mg/ml; NAD bzw. cNAD 50 mg/ml; Puffer: 75 mM Na₄P₂O₇, Glycin, pH 9,0; Temperatur 35 °C.

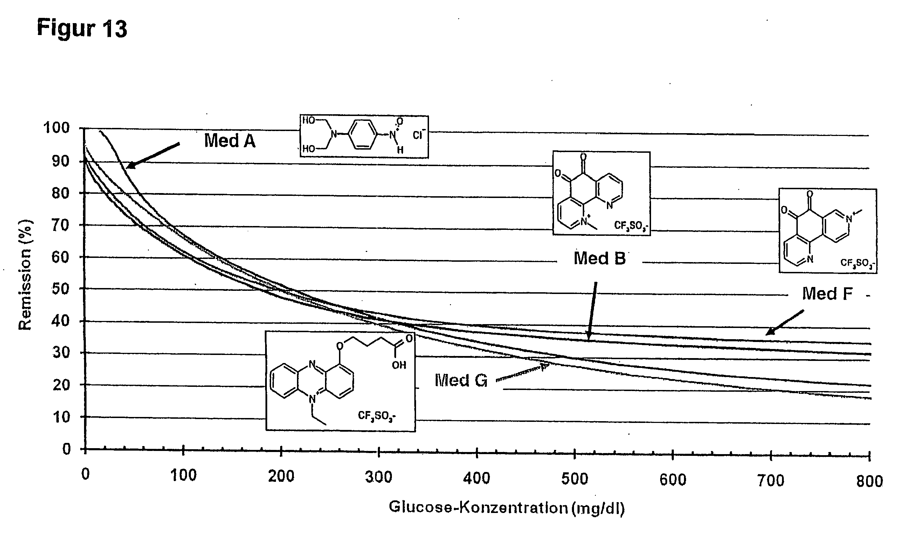

**Figur 13****:** Darstellung verschiedener Funktionskurven von Glucose-Dehydrogenase nach 11 Wochen Lagerung in Gegenwart von NAD und unterschiedlicher Mediatoren bei Raumtemperatur.

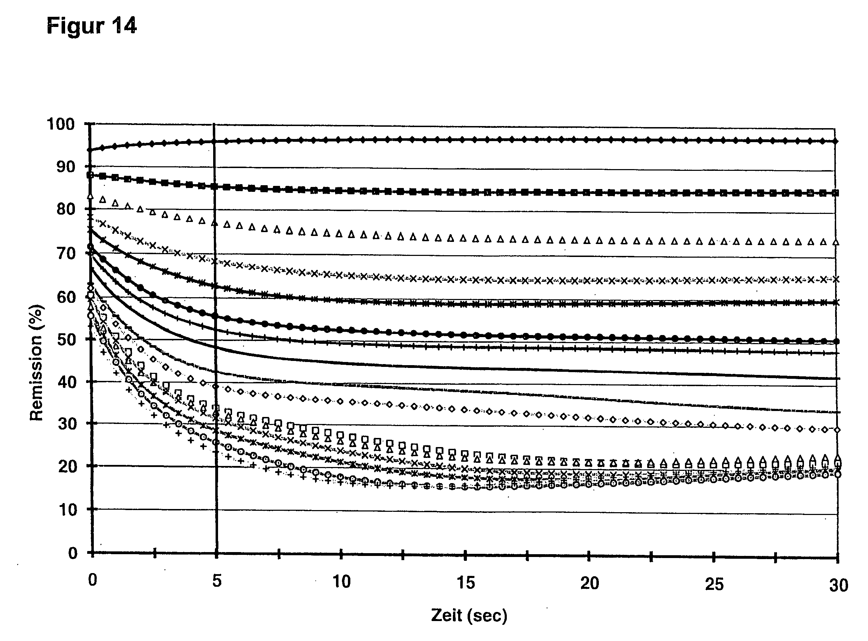

**Figur 14****:** Darstellung der Ergebnisse der Enzymkinetik von Glucose-Dehydrogenase in Gegenwart von NAD und 1-(3-Carboxypropoxy)-5-ethylphenazinium-trifluormethansulfonat bei verschiedenen Glucose-Konzentrationen.

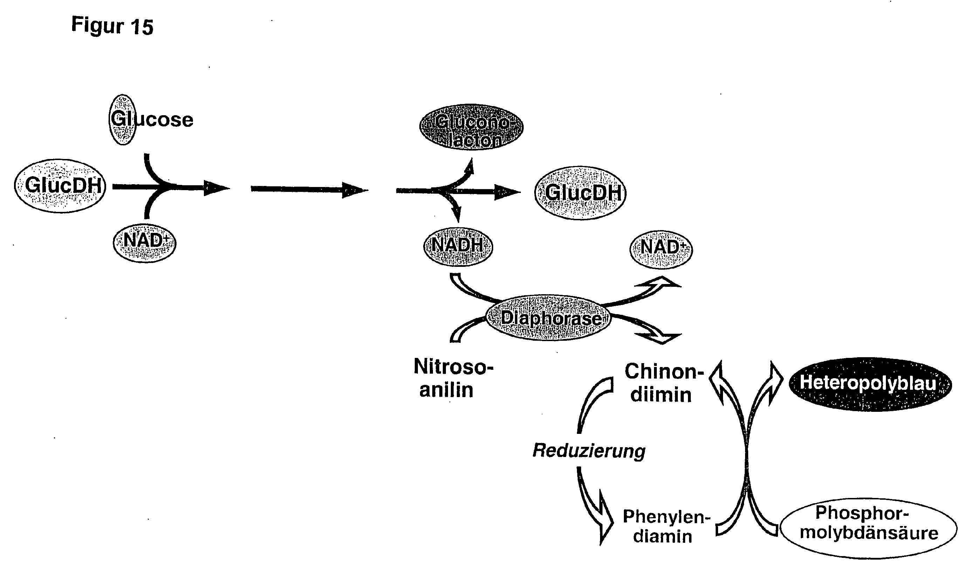

**Figur 15****:** Schematische Darstellung des Glucose-Nachweises mit GlucDH als Enzym und Diaphorase als Mediator.

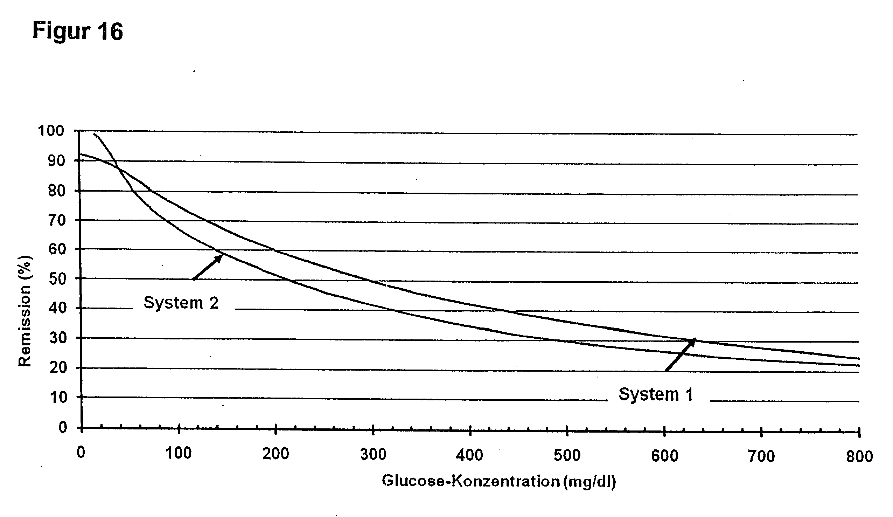

**Figur 16****:** Darstellung der Funktionskurven von Glucose-Dye-Oxidoreduktase (GlucDOR) in Gegenwart von Pyrrolochinolinchinon (PQQ) und [(4-Nitrosophenyl)imino]dimethanol-Hydrochlorid als Mediator bzw. von Glucose-Dehydrogenase in Gegenwart von NAD und Diaphorase/[(4-Nitrosophenyl)imino]dimethanol-Hydrochlorid als Mediator.

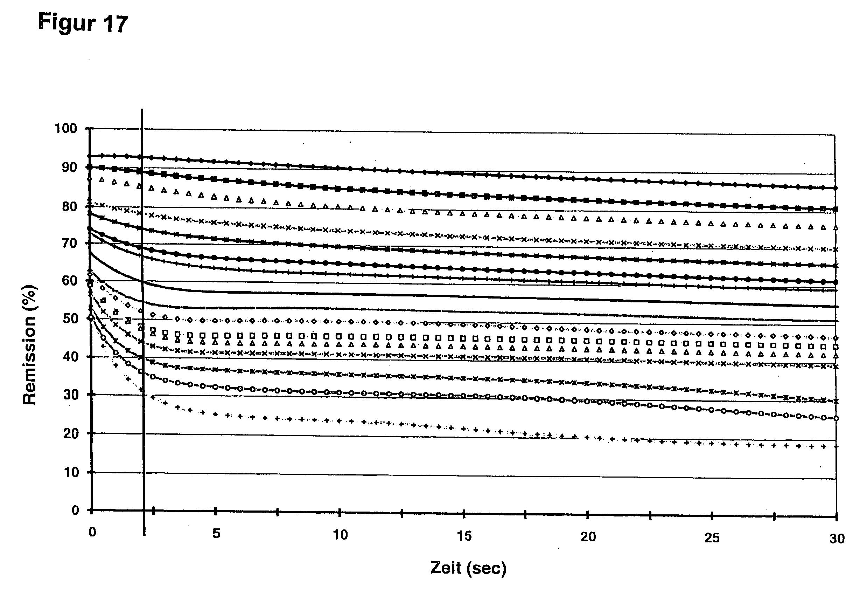

**Figur 17****:** Darstellung der Ergebnisse der Enzymkinetik von Glucose-Dehydrogenase in Gegenwart von NAD und Diaphorase bei verschiedenen Glucose-Konzentrationen.

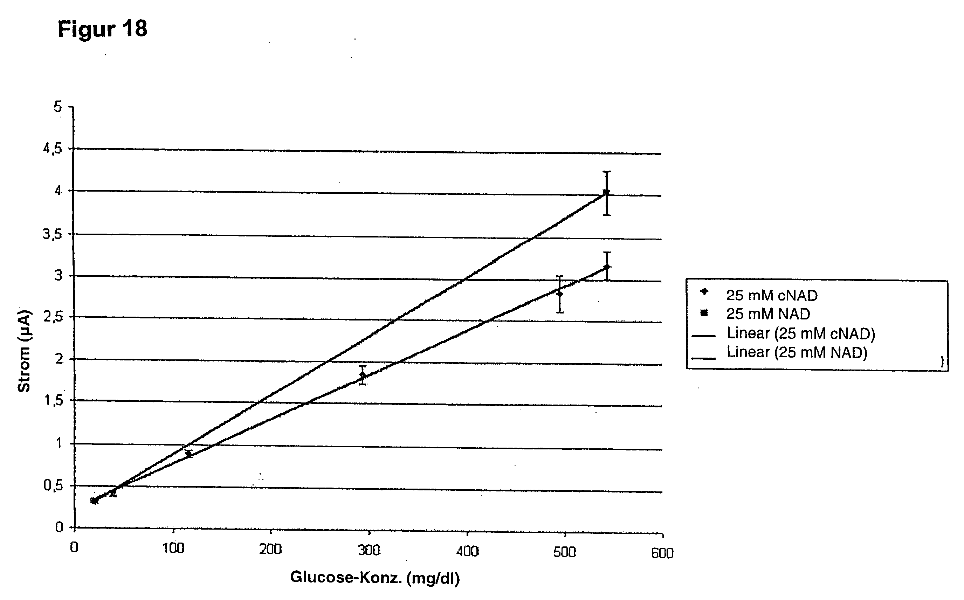

**Figur 18****:** Darstellung des in Abhängigkeit von der Glucose-Konzentration gemessenen Stromes bei elektrochemischer Bestimmung von Glucose unter Verwendung von Glucose-Dehydrogenase in Gegenwart von NAD bzw. cNAD. Testbedingungen: 25 mM NAD bzw. cNAD; 2.5 Sekunden Verzögerung; 5 Sekunden Messzeit.

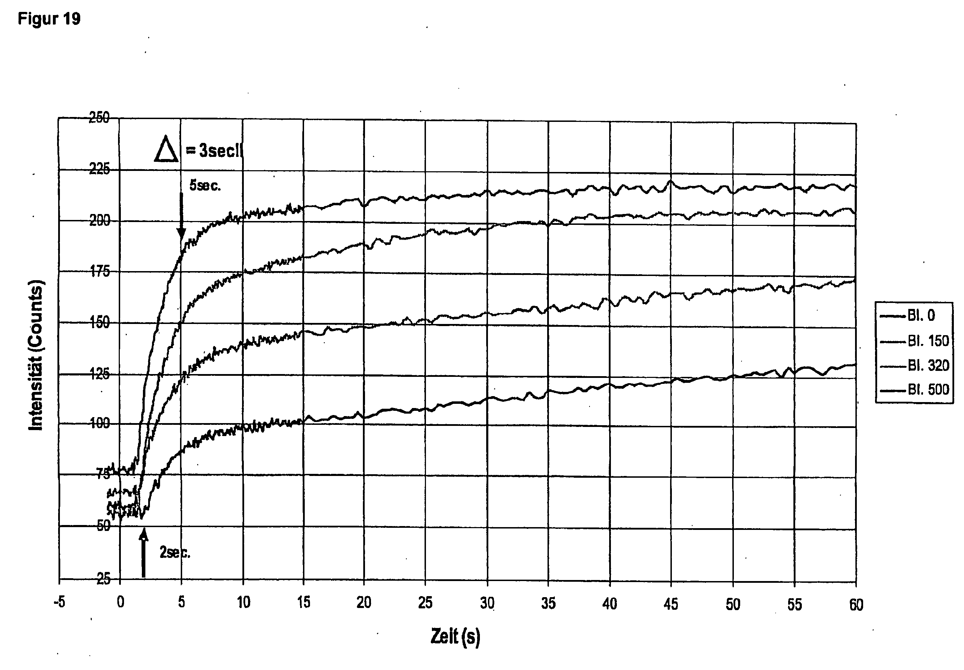

**Figur 19****:** Darstellung verschiedener Funktionskurven von Glucose-Dehydrogenase in Gegenwart von NAD nach Einstrahlung von UV-Licht mit einer Wellenlänge von 360 nm.

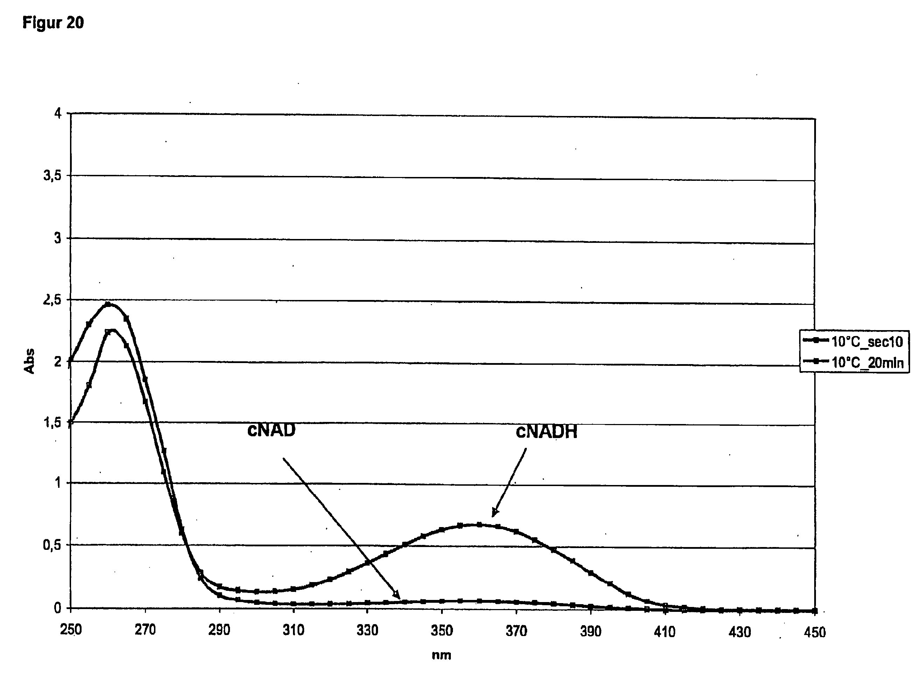

**Figur 20****:** Darstellung des Absorptionsspektrums von cNAD und cNADH im Wellenlängenbereich von 250-450 nm.

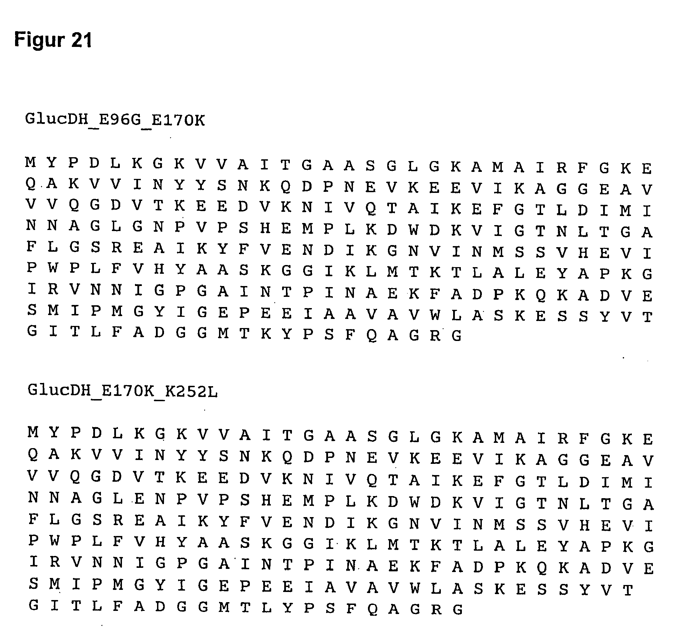

**Figur 21****:** Darstellung der Aminosäuresequenzen der Glucose-Dehydrogenase-Doppelmutanten GlucDH_E96G_E170K und GlucDH_E170K_K252L.

### Beispiel 1

Der Glucose-spezifischen GlucDH wurden carbaNAD (Fig. 1A) oder NAD zugesetzt. Diese Rezepturen wurden jeweils auf Pokalonfolie (Lonza) aufgetragen und nach Trocknung unter warmen und feuchten Bedingungen (32°C, 85% relative Luftfeuchtigkeit) eingelagert. Anschließend wurden in regelmäßigen Abständen die Reaktionskinetik sowie die Funktionskurve bestimmt. Parallel wurde zu den jeweiligen Messterminen eine cNAD/NAD-Analytik sowie eine Bestimmung der Restaktivität des Enzyms vorgenommen.

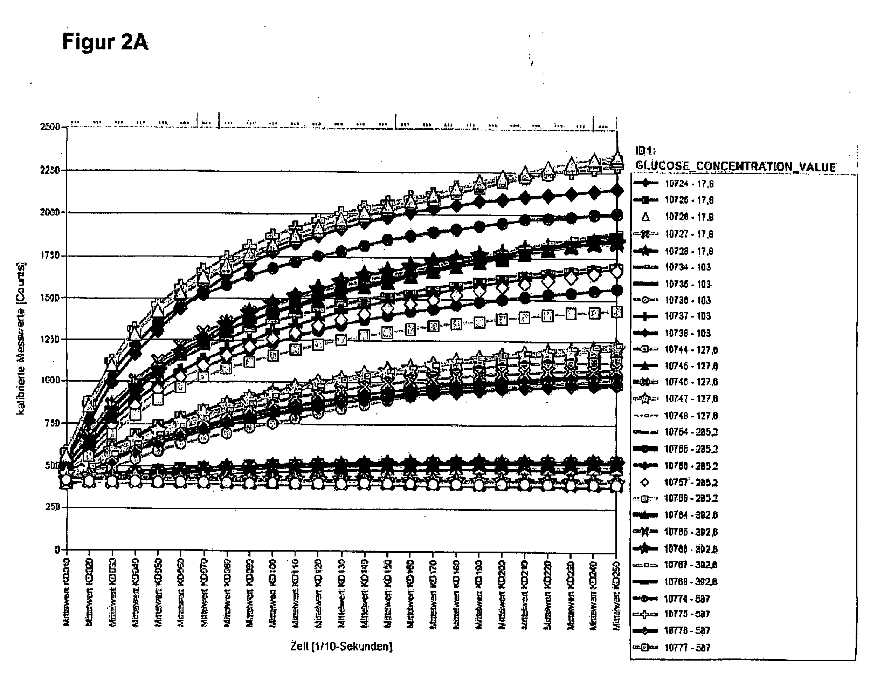

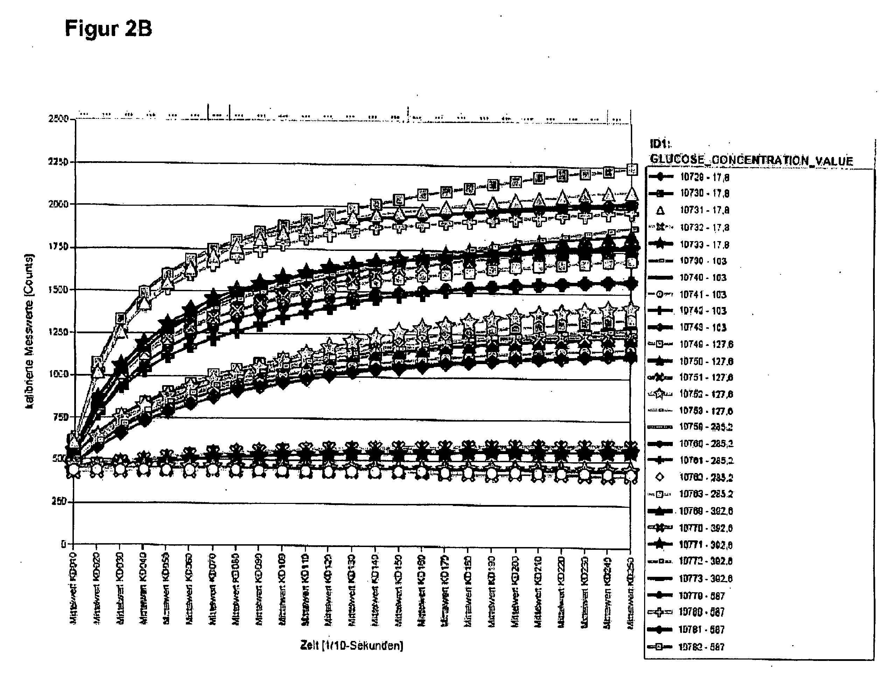

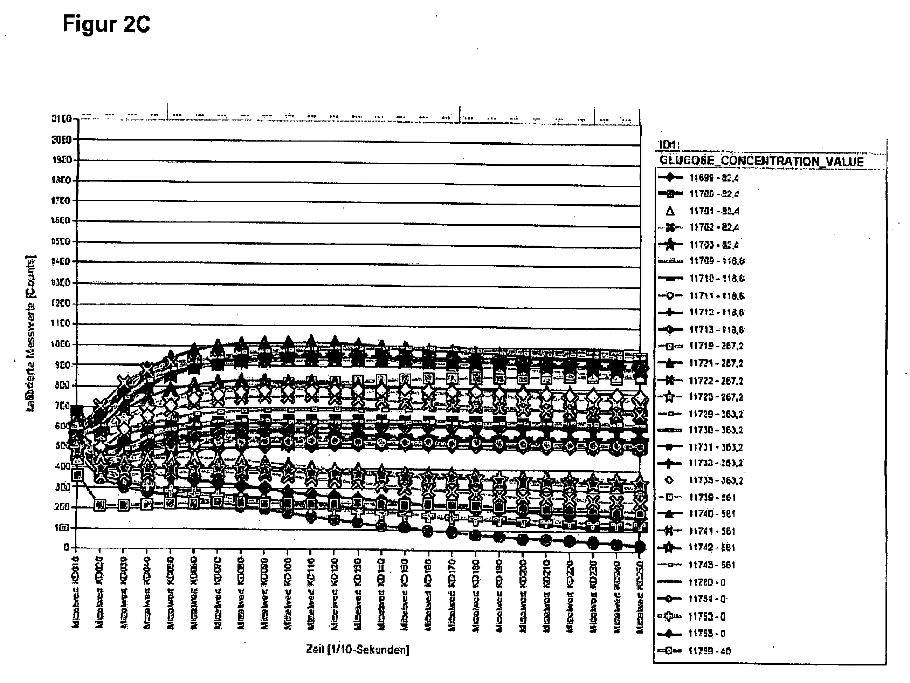

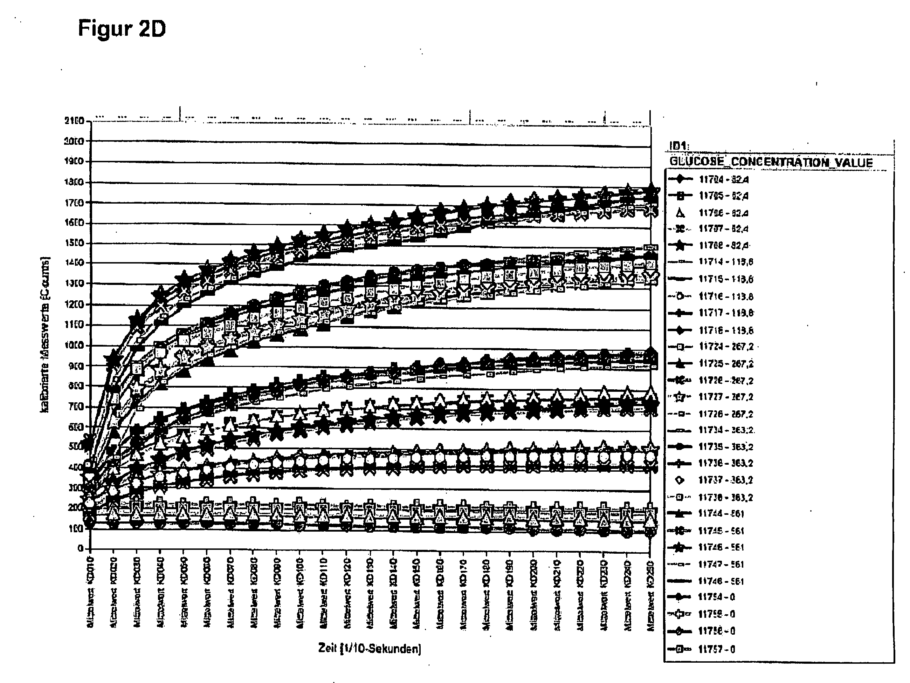

Die am ersten Tag bestimmten Kinetik-Kurven für NAD (Figur 2A) und cNAD (Figur 2B) sind vergleichbar und zeigen auch einen ähnlichen Hub in der Glucoseabhängigkeit. Nach 5 Wochen ist jedoch ein deutlicher Unterschied in den Kinetik-Kurven zu erkennen. Während die Kinetik für NAD (Figur 2C) stark in ihrer Dynamik nachlässt, bleibt die Kinetik des mit cNAD stabilisierten Enzyms praktisch unverändert (Figur 2D).

Auch in den Blankwerten (Trockenleerwert vor Auftrag einer Blutprobe) zeigt sich ein deutlicher Unterschied, wie aus Figur 3 ersichtlich ist. Der Anstieg des Trockenleerwerts bei NAD ist auf die Bildung von fluoreszierenden Teilchen zurückzuführen (Oppenheimer (1982), Supra). Überraschenderweise geschieht dies nicht bei cNAD.

Die unterschiedliche Stabilität von Glucose-Dehydrogenase in Gegenwart von NAD bzw. cNAD ist auch aus einem Vergleich der Figuren 4 und 5 ersichtlich. Nach 5 Wochen liegt die Funktionskurve bei dem mit cNAD stabilisierten Enzym noch in der Schar der voherigen Messungen (Figur 5A), während bei dem mit NAD versetzten Enzym (Figur 4) die Kurve bei höheren Konzentrationen abknickt, was ein typisches Zeichen für zu geringe Mengen an Enzym/Coenzym ist. Figur 5B zeigt verschiedene Funktionskurven der mit cNAD stabilisierten Glucose-Dehydrogenase über einen Zeitraum von 24 Wochen. In diesem Zusammenhang wird deutlich, dass sich die Funktion des Enzyms bei hohen Glucose-Konzentrationen über den gesamten Zeitraum hinweg nur geringfügig verändert und nach 24 Wochen etwa dem nach 5 Wochen erhaltenen Wert entspricht.

Die Beziehung zwischen Struktur des Coenzyms und dessen Stabilität über einen vorbestimmten Zeitraum ergibt sich aus Figur 6. Demnach beträgt der Restgehalt an cNAD in einem Glucose-Nachweisreagenz nach 24 Wochen Lagerung (bei 32°C und 85% relativer Luftfeuchtigkeit) noch etwa 80% des Ausgangswertes, während sich der Gehalt an NAD in einem mit NAD stabilisierten Glucose-Nachweisreagenz bereits nach 5 Wochen auf etwa 35% des Ausgangswertes verringert und gemäß Extrapolation nach etwa 17 Wochen auf null reduziert.

Vollkommen überraschend ist das Ergebnis der Restaktivitätsbestimmung des aktiven Enzyms GlucDH nach 5 Wochen bei 32°C und 85% relative Luftfeuchtigkeit (Figur 7A). Das mit NAD stabilisierte Enzym weist nur noch eine extrem geringe Enzymaktivität auf (0,5%), während das mit cNAD stabilisierte Enzym noch eine Restaktivität von 70% besitzt (jeweils im Vergleich zu den mit Trockenmittel (TM) im Kühlschrank (KS) eingelagerten Proben). Nach 24 Wochen bei 32°C und 85% relativer Luftfeuchtigkeit (Figur 7B) beträgt die Restaktivität des Enzyms bei Stabilisierung mit cNAD noch immer etwa 25%.

Wird anstelle des Wildtyp-Enzyms (aus Bacillus subtilis) eine Mutante verwendet, so kann die Restaktivität von GlucDH noch weiter gesteigert werden. Nach 24 Wochen Lagerung bei 32°C und 85% relativer Luftfeuchtigkeit in Gegenwart von cNAD beträgt die Restaktivität einer Mutante GlucDH_E96G_E170K mit Aminosäuresubstitutionen Glutaminsäure glycin an Position 96 und Glutaminsäure → Lysin an Position 170 (GlucDH-Mut1) des Wildtyp-Enzyms etwa 70%, während die Restaktivität einer Mutante GlucDH_E170K_K252L mit Aminosäuresubstitutionen Glutaminsäure → Lysin an Position 170 und Lysin → Leucin an Position 252 (GlucDH-Mut2) bei etwa 50% liegt (Figur 8).

Auch eine Lagerung von Glucose-Dehydrogenase in flüssiger Phase zeigt deutlich den Unterschied zwischen NAD bzw. cNAD (Figuren 9A und 9B). Nach 95 Stunden bei 50°C liegt die Restaktivität von Glucose-Dehydrogenase in Gegenwart des nativen Coenzyms NAD bei» 5%, während die Restaktivität der GlucDH in Gegenwart des artifiziellen Coenzyms cNAD bei 75% liegt (Figur 9A). Nach 336 Stunden Lagerung bei 50°C beträgt die Restaktivität des mit NAD stabilisierten Enzyms nur mehr etwa 1%; für das in Gegenwart von cNAD gelagerte Enzym wird eine Restaktivität von immer noch etwa 70% beobachtet. Die entsprechenden SDS-Gele zeigen ebenfalls eine Veränderung der GlucDH-Bande in Gegenwart des nativen Coenzyms NAD: es zeigen sich neue Banden bei höheren Molmassen und eine Verschiebung der 30 kDa-Bande.

Insgesamt ist es ein höchst überraschendes Ergebnis, dass die Stabilisierung des Cofaktors gleichzeitig eine Stabilisierung des Enzyms bewirkt - und dies nicht allein durch den kooperativen Effekt des besseren Zusammenhaltes des Enzyms. Der Zerfall des Cofaktors NAD hat einen negativen Effekt auf die Stabilität des Enzyms GlucDH und beschleunigt sogar dessen Inaktivierung. Der Austausch von nativem NAD durch künstliche Analoga erlaubt eine Lagerung der GlucDH unter Stressbedingungen (z. B. erhöhte Temperatur) auch in Gegenwart eines Cofaktors.

Mit einem solchen System lassen sich Blutglucose-Teststreifen mit erheblich verbesserten Stabilitätseigenschaften generieren, bei denen eine Darreichungsform ohne Trockenmittel möglich ist.

### Beispiel 2

Einer Alkohol-Dehydrogenase umfassenden Nachweislösung wurden cNAD oder NAD zugesetzt. Diese Mischungen wurden bei 2-8°C bzw. bei 35°C eingelagert. Anschließend wurde in regelmäßigen Abständen die Stabilität von Alkohol-Dehydrogenase (ADH) überprüft und eine Bestimmung der Restaktivität des Enzyms vorgenommen.

Figur 10 zeigt die Linearität der Umsetzung von Ethanol mittels Alkohol-Dehydrogenase (ADH) in Gegenwart verschiedener Konzentrationen an cNAD, was die praktische Anwendbarkeit des Enzymsystems ADH/cNAD zur Bestimmung von Ethanol belegt. Aus den Kinetik-Kurven der Umsetzung von Ethanol mittels einer Kombination von Alkohol-Dehydrogenase und cNAD ergibt sich zudem eine deutliche Abhängigkeit von der Konzentration des Substrats, wobei sich die Geschwindigkeit der Umsetzung mit steigender Ethanol-Konzentration erhöht (Figur 11).

Wiederum zeigt eine Lagerung in flüssiger Phase den Unterschied zwischen einer Lagerung in Anwesenheit von NAD bzw. cNAD (Figur 12). Nach 65 Stunden bei 35°C liegt die Restaktivität von Alkohol-Dehydrogenase in Gegenwart des nativen Coenzyms NAD bei etwa 6%, während die Restaktivität des Enzyms in Gegenwart des artifiziellen Coenzyms cNAD noch bei etwa 60% liegt.

Wird Alkohol-Dehydrogenase zusammen mit nativem NAD bzw. mit cNAD über mehrere Monate bei 2-8°C im Kühlschrank gelagert, so beobachtet man im Falle von cNAD über die Lagerungsdauer hinweg eine deutlich geringere Abnahme der Enzymaktivität. Während der Unterschied nach 2-wöchiger Lagerung noch als gering einzustufen ist, ergibt sich für Alkohol-Dehydrogenase nach 12-monatiger Lagerung in Gegenwart von 16 mM cNAD eine etwa 20% höhere Restaktivität im Vergleich zu einer entsprechenden Lösung mit 16 mM NAD als Coenzym. Die Ergebnisse sind in Tabelle 1 dargestellt.

**Tabelle 1**

| Einsatzstoff | Konz. (mmol /l) | U/ml (Startwert) | % zu Einwaage (Startwert) | 2 Wochen bei 2-8°C (% zu Start) | 3 Monate bei 2-8°C (% zu Start) | 7.5 Monate bei 2-8°C (% zu Start) | 10 Monate bei 2-8°C (% zu Start) | 12 Monate bei 2-8°C (% zu Start) |
|---|---|---|---|---|---|---|---|---|
| Vergleichsansatz | 16 (NAD) | 142.2 | 85.2 | 96.9 | 92.1 | 80.2 | 70.3 | 60.5 |
| Austausch NAD/cNAD | 16 | 120.2 | 71.9 | 100.7 | 91.3 | 95.6 | 88.2 | 83.8 |

Das Ausmaß der Stabilisierung von der Menge an verwendetem cNAD ist aus Tabelle 2 ersichtlich. Demzufolge kann in Proben, welche für 2 Wochen bei 2-8°C gelagert werden, die Restaktivität von Alkohol-Dehydrogenase durch Steigerung der Konzentration an cNAD etwas erhöht werden. Im Stressmodell, welches eine Lagerung des Enzyms für 2 Wochen bei 35°C vorsieht, kann die Abnahme der Enzymaktivität von Alkohol-Dehydrogenase durch steigende Konzentrationen an cNAD indessen signifikant erhöht werden, wobei im Falle einer Konzentration von 15 mM an cNAD eine etwa 45% höhere Restaktivität im Vergleich zu einer Lösung des Enzyms in Gegenwart von 0.5 mM cNAD beobachtet wird.

**Tabelle 2**

| Probe | pH/Zusatz | U/ml (Startwert) | % zu Einwaage (Startwert) | 2 Wochen bei 2-8°C (% zu Start) | 2 Wochen bei 35°C (% zu Start) |
|---|---|---|---|---|---|
| 1 | pH 7.85 0.5 mM cNAD | 123.1 | 73.7 | 93.8 | 31.1 |
| 2 | pH 7.85 1 mM cNAD | 125.8 | 75.3 | 97.7 | 45.5 |
| 3 | pH 7.85 5 mM cNAD | 125.1 | 74.9 | 101.1 | 74.1 |
| 4 | pH 7.85 15 mM cNAD | 123.7 | 74.1 | 104.0 | 77.6 |

### Beispiel 3

Zur Bestimmung von Glucose wurden verschiedene Testsysteme, welche jeweils Glucose-Dehydrogenase, NAD, einen Mediator und ggf. einen optischen Indikator umfassten, photometetrisch bzw. elektrochemisch vermessen.

Für photometetrische Messungen wurden zunächst vier Testelemente, welche jeweils 11 Wochen bei Raumtemperatur gelagert worden waren und neben Glucose-Dehydrogenase, NAD und einem Mediator 2,18-Phosphormolybdänsäure enthielten, bei verschiedenen Glucose-Konzentrationen untersucht.

Wie aus Figur 13 ersichtlich ist, wurde bei allen vier eingesetzten Mediatoren, d.h. [(4-Nitrosophenyl)imino]dimethanol-Hydrochlorid (Med A), 1-Methyl-5,6-dioxo-5,6-dihydro-1,10-phenanthrolinium-trifluormethansulfonat (Med B), 7-Methyl-5,6-dioxo-5,6-dihydro-1,7-phenanthrolinium-trifluor-methansulfonat (Med F) und 1-(3-Carboxypropoxy)-5-ethylphenazinium-trifluormethansulfonat (Med G), mit steigender Glucose-Konzentration ein Abfall der Remission beobachtet, womit oben genannte Mediatoren grundsätzlich für eine Bestimmung von Glucose mittels Photometrie geeignet sind.

Bei hohen Glucose-Konzentrationen im Bereich von 800 mg/dl beträgt die Remission der vermessenen Probe bei Verwendung von [(4-Nitrosophenyl)imino]dimethanol-Hydrochlorid bzw. 1-(3-Carboxypropoxy)-5-ethylphenazinium-trifluormethansulfonat noch etwa 20%, was auf die besondere Eignung dieser beiden Mediatoren für photometrische Messungen unter Verwendung des Systems Glucose-Dehydrogenase/NAD, und damit auch des Systems Glucose-Dehydrogenase/cNAD, schließen lässt. Die Kinetik der Umsetzung von Glucose mittels des Systems Glucose-Dehydrogenase, NAD, 1-(3-Carboxypropoxy)-5-ethylphenazinium-trifluormethansulfonat und 2,18-Phosphormolybdänsäure bei Glucose-Konzentrationen im Bereich von 0 bis 800 mg/dl ist in Figur 14 dargestellt.

Wie der schematischen Darstellung in Figur 15 zu entnehmen ist, kann die photometrische Bestimmung von Glucose auch unter (zusätzlicher) Verwendung von Diaphorase als Zwischenmediator erfolgen. Figur 16 zeigt für das System Glucose-Dehydrogenase, NAD, Diaphorase, [(4-Nitrosophenyl)imino]dimethanol-Hydrochlorid und 2,18-Phosphormolybdänsäure (System 1) einen konzentrationsabhängigen Abfall der Remission. Als Vergleich diente das System Glucose-Dye-Oxidoreduktase, Pyrrolochinolinchinon, [(4-Nitrosophenyl)imino]dimethanol-Hydrochlorid und 2,18-Phosphormolybdänsäure (System 2), welches gleichermaßen einen konzentrationsabhängigen Abfall der Remission bedingt, aufgrund der geringen Spezifität von Glucose-Dye-Oxidoreduktase jedoch Nachteile besitzt. Die Kinetik der Umsetzung von Glucose mittels des Systems 1 bei Glucose-Konzentrationen im Bereich von 0 bis 800 mg/dl ist in Figur 17 abgebildet.

Alternativ zur Photometrie kann zum Zwecke der Analytbestimmung auch eine elektrochemische Messung eingesetzt werden. So zeigte sich sowohl für ein Testelement, welches neben Glucose-Dehydrogenase NAD als Coenzym und 1-(3-Carboxypropoxy)-5-ethylphenazinium-trifluormethansulfonat als Mediator enthielt, als auch für ein korrespondierendes System, welches anstelle von NAD das stabilisierte Coenzym cNAD umfasste, eine lineare Abhängigkeit des zur Reoxidation des reduzierten Mediators benötigten Stromes von der Glucose-Konzentration (Figur 18).

Damit ist gezeigt, dass die Analytbestimmung unter Verwendung des Systems Dehydrogenase/stabilisiertes Coenzym auch mittels elektrochemischer Detektion und Auswertung mit einer anderen Wellenlänge, welche unabhäbgig vom Coenzym ist, erfolgen kann. Durch den Einsatz des stabilisierten Enzym/Coenzym-Paares sollte die Gesamtrezeptur auch weitergehend stabilisiert werden.

### Beispiel 4

Zur Bestimmung ihrer Stabilität gegenüber Umgebungslicht wurden verschiedene Testsysteme, welche jeweils ein Enzym ausgewählt aus Glucose-Dehydrogenase (GlucDH), Glucose-6-phosphat-Dehydrogenase (G6PDH) und Glucose-Dehydrogenase-Mutante2 (GlucDH-Mut2) in Kombination mit NAD oder carbaNAD enthielten, in Anlehnung an das in WO 03/097859 A2 beschriebene Verfahren hergestellt und anschließend mit einer Blutprobe versetzt. Im einzelnen erfolgte die Herstellung der Testsysteme durch Aufbringen einer Enzym und Coenzym umfassenden photopolymerisierbaren flüssigen Zusammensetzung auf einen Träger und anschließendes Bestrahlen der Zusammensetzung mit Licht einer Wellenlänge von 360 nm und 400 W für zehn Sekunden, wobei Reagenzienschichten mit einer Dicke von 12-15 µm erhalten wurden. Die Detektion erfolgte im Fluoreszenzmodus über eine Dauer von einigen Minuten.

Figur 19 zeigt die Messergebnisse für das System GlucDH/NAD. Wie aus der Grafik hervorgeht, wird über die gesamte Messdauer keinerlei Verringerung der Fluoreszenz beobachtet, was darauf schließen lässt, dass selbst minutenlanges Bestrahlen der Reagenzienschicht mit energiereicher Strahlung aufgrund mangelnder Absorption des Systems GlucDH/NAD im Bereich ≥ 300 nm keine Beeinträchtigung der Reagenzienschicht hervorruft. Unter Berücksichtigung dessen, dass auch das System G6PDH/NAD sowie die carbaNAD-enthaltenden Systeme GlucDH/carbaNAD, G6PDH/carbaNAD und GlucDH-Mut2/carbaNAD im Wellenlängenbereich ≥ 300 nm nicht absorbieren (Figur 20), weisen auch diese Systeme allesamt eine hohe Stabilität gegenüber Umgebungslicht auf (Ergebnisse nicht dargestellt).

### Beispiel 5

Nachfolgend sind konkrete Beispiele für Zusammensetzungen angegeben, welche zur Herstellung der hierin beschriebenen diagnostischen Testelemente verwendet werden können.

### a) Flüssigreagenz zur Bestimmung der Aktivität von Lactat-Dehydrogenase

Zur Bestimmung der Aktivität von Lactat-Dehydrogenase wurde ein Flüssigreagenz angewendet, welches u.a. Diaphorase, carbaNAD, ein Tetrazoliumsalz und Lactat umfasst. Das Nachweisreagenz, welches bei 25°C in Lösung gelagert wurde, enthält die folgenden Inhaltsstoffe:
3 U/ml Diaphorase (aus Schweineherz)
2 mM carbaNAD oder 0.2 mM NAD
2 mM Tetrazoliumsalz WST-3
50 mM Na-Lactat
0.1 M Tricin/HCl, pH 8.8

Eine Messung der Aktivität von Diaphorase zu verschiedenen Lagerungszeitpunkten zeigte eine deutlich erhöhte Restaktvität der carbaNAD-enthaltenden Rezeptur im Vergleich zu einer korrespondierenden Rezeptur mit NAD als Coenzym.

### b) Teststreifen zur Bestimmung von Blutglucose

Zur Blutglucosebestimmung wurde eine Zusammensetzung verwendet, welche u.a. Glucose-Dehydrogenase (GlucDH), carbaNAD, Diaphorase, Nitrosoanilin sowie Phosphormolybdänsäure umfasst. Durch Aufbringen einer ersten Rezeptur auf eine Polycarbonatfolie mittels eines Rakels (Schichthöhe 100 µm), Trocknen der ersten Schicht, Aufbringen einer zweiten Rezeptur auf die erste Schicht (Rakelspalt 30 µm), und Trocknen der zweiten Schicht wurden Teststreifen erhalten, welche bei 32°C und 30-70% relativer Luftfeuchtigkeit gelagert wurden. Die für die erste und zweite Schicht verwendeten Rezepturen sind in Tabelle 3 dargestellt:

**Tabelle 3**

| Inhaltsstoffe | 1. Schicht | 2. Schicht |
|---|---|---|
| Xanthan Gum | 0.09 g | ----- |
| Natrium-Aluminiumsilikat | 5.00 g | ----- |
| Polyvinylpropionat-Dispersion (50 Gew.% in Wasser) | 4.80 g | 5.80 g |
| Methylvinylether-Maleinsäure-Copolymer | ----- | 1.40 g |
| Titandioxid | ----- | 14.00 g |
| NaOH 16% | ----- | 2.80 g |
| Kieselsäure | ----- | 2.50 g |
| Phosphatpuffer 0.1 M | 13.80 g | 4.40 g |
| N-Octanoyl-N-methyl-glucamid | 0.17 g | 0.34 g |
| Na-methyl-oleyltaurat | 0.03 g | 0.03 g |
| Polyvinylpyrrolidon (MG 25000) | 0.85 g | 1.80 g |
| Tetraethylammoniumchlorid | 0.12 g | ----- |
| Bis-(2-hydroxyethyl)-(4-hydroxyiminocyclohexa-25-dienylidin)-ammoniumchlorid | 0.10 g | ----- |
| 2,18-Phosphormolybdänsäure-Hexanatriumsalz | 0.50 g | ----- |
| NaCl | 1.00 g | ----- |
| carbaNAD (alternativ NAD) | 1.00 g (0.10 g) | ----- |
| Diaphorase | 1.94 g (203 KU) | ----- |
| Glucose-Dehydrogenase | 0.97 g (207 KU) | ----- |
| Hexanol | 0.17 g | 0.16 g |
| 2-Methoxy-propanol | 4.30 g | 4.30 g |

Eine Messung der Aktivität von Diaphorase wurde zu verschiedenen Lagerungszeitpunkten durch Extraktion des Enzyms aus dem Teststreifen vorgenommen, wobei im Falle der carbaNAD-enthaltenden Rezeptur eine deutlich erhöhte Restaktvität im Vergleich zur NAD-enthaltenden Rezeptur beobachtet wurde.

### c) Teststreifen zur Bestimmung von Triglyceriden

Zur Bestimmung von Triglyceriden wurde eine Zusammensetzung eingesetzt, welche u.a. Glycerin-Dehydrogenase, carbaNAD, Diaphorase und ein Tetrazoliumsalz umfasst. Durch Aufbringen der in Tabelle 4 genannten Rezeptur auf eine Polycarbonatfolie mittels eines Rakels (Schichthöhe 80 µm) und anschließendes Trocknen wurden Teststreifen erhalten, die bei 32°C und 30-70% relativer Luftfeuchtigkeit gelagert wurden.

**Tabelle 4**

| Inhaltsstoffe | Menge |
|---|---|
| Xanthan Gum | 0.30 g |
| Kieselsäure (Aerosil) | 5.00 g |
| Polyvinylpropionat-Dispersion (50 Gew.% in Wasser) | 6.00 g |
| Titandioxid | 18.00 g |
| N-Octanoyl-N-methyl-glucamid | 0.20 g |
| Polyvinylpyrrolidon (MG 25000) | 0.40 g |
| Tricin | 0.30 g |
| HCl | ad pH 8.5 |
| Lipase (Schweinepankreas), 12 KU/g | 5.00 g |
| Glycerin-Dehydrogenase, 100 U/mg | 1.00 g |
| carbaNAD (alternativ NAD) | 1.60 g (0.20 g) |
| Diaphorase, 20 U/mg | 2.00 g |
| WST-5 | 2.00 g |
| Wasser | 82.00 g |

Eine Messung der Aktivität von Diaphorase wurde zu verschiedenen Lagerungszeitpunkten durch Extraktion des Enzyms aus dem Teststreifen vorgenommen, wobei im Falle der carbaNAD-enthaltenden Rezeptur eine deutlich erhöhte Restaktvität im Vergleich zur NAD-enthaltenden Rezeptur beobachtet wurde.

### Beispiel 6

Zur Evaluierung der Stabilisierung von Glucose-Dehydrogenase bzw. Diaphorase durch carbaNAD wurden in Analogie zu Beispiel 5 eine Reihe von Teststreifen hergestellt. Die für die erste und zweite Schicht der Teststreifen verwendeten Rezepturen sind in Tabelle 5 dargestellt:

**Tabelle 5**

| Inhaltsstoffe | 1. Schicht | 1. Schicht | 2. Schicht |
|---|---|---|---|
| Xanthan Gum | 0.29 g | 0.29 g | ----- |
| Gantrez S97 | ----- | ----- | 1.44 g |
| Tetraethylammoniumchlorid | 0.07 g | 0.07 g | 0.45 g |
| Mega 8 | 0.17 g | 0.17 g | 0.36 g |
| Geropon T77 | 0.03 g | 0.03 g | 0.03 g |
| Polyvinylpyrrolidon (MG 25000) | 0.87 g | 0.87 g | 1.99 g |
| Transpafill (alternativ Kieselsäure) | 4.82 g (4.79 g) | 4.82 g (4.79 g) | ----- |
| Kieselsäure | ----- | ----- | 16.02 g |
| Titandioxid | ----- | ----- | 2.18 g |
| Propiofan | 5.04 g | 5.02 g | 6.14 g |
| N, N-bis-(2-hydroxyethyl)-4-nitrosoanilin | 0.10 g | 0.10 g | 0.16 g |
| 2,18-Phosphormolybdänsäure-Hexanatriumsalz | 0.33 g | 0.33 g | 1.98 g |
| K/Na-Puffer 0.1 M | 10.00 g | 9.96 g | ----- |
| NAD | 0.58 g | ----- | ----- |
| carbaNAD | ----- | 4.00 g | ----- |
| Glucose-Dehydrogenase, Mutante 2 | 1.06 g (324 KU) | 1.06 g (324 KU) | ----- |
| Diaphorase | 0.78 g (80 KU) | 1.26 g (130 KU) | ----- |
| K₃[Fe(CN)₆] | 0.01 g | 0.01 g | 0.01 g |
| NaOH 16% | 0.25 g | 0.28 g | 2.69 g |
| tert. Amylalkohol | 1.00 g | 1.00 g | 1.07 g |

Die Lagerung der Teststreifen erfolgte über einen Zeitraum von 18 Wochen bei Temperaturen von 5°C (KS), 25°C (RT), 30°C (GT), 35°C (DT) bzw. 45°C (HT), wobei zu Beginn der Lagerung (0 Wochen), nach 6 Wochen, nach 9 Wochen, nach 12 Wochen sowie nach 18 Wochen die Enzymaktivität in den Teststreifen bestimmt wurde. Die Ergebnisse sind in Tabelle 6 (Glucose-Dehydrogenase) bzw. in Tabelle 7 (Diaphorase) dargestellt:

**Tabelle 6**

| Lagerungstemperatur | Coenzym | Enzymaktivität (U/cm²) | | | | | |
|---|---|---|---|---|---|---|---|
| | | 0 Wochen | 6 Mochen | 9 Wochen | 12 Wochen | 18 Wochen | Soll |
| KS | NAD | 14.6 | 16.5 | 16.6 | 14.4 | 13.8 | 25.4 |
| RT | | | 15.8 | 14.4 | 12.9 | 12.2 | |
| GT | | | n.b. | n.b. | 9.7 | 9.1 | |
| DT | | | 12.9 | 10.9 | 9.4 | 8.5 | |
| HT | | | 7.8 | 6.2 | n.b. | n.b. | |
| KS | carbaNAD | 19.8 | 20.4 | 21.2 | 18.5 | 18.8 | 25.8 |
| RT | | | 20.7 | 21.5 | 18.0 | 17.7 | |
| GT | | | n.b. | n.b. | 17.6 | 16.8 | |
| DT | | | 19.8 | 20.2 | 17.2 | 16.9 | |
| HT | | | 19.0 | 18.8 | n.b. | n.b. | |

**Tabelle 7**

| Lagerungstemperatur | Coenzym | Enzymaktivität (U/cm²) | | | | | |
|---|---|---|---|---|---|---|---|
| | | 0 Wochen | 6 Mochen | 9 Wochen | 12 Wochen | 18 Wochen | Soll |
| KS | NAD | 2.7 | 2.2 | 2.4 | 3.3 | 3.7 | 6.3 |
| RT | | | 2.3 | 2.2 | 2.8 | 3.4 | |
| GT | | | n.b. | n.b. | 2.4 | 2.8 | |
| DT | | | 1.5 | 1.8 | 2.4 | 2.8 | |
| HT | | | 1.5 | 1.1 | n.b. | n.b. | |
| KS | carbaNAD | 4.6 | 4.4 | 5.2 | 4.8 | 7.0 | 10.4 |
| RT | | | 4.5 | 5.1 | 4.6 | 6.7 | |
| GT | | | n.b. | n.b. | 4.3 | 6.2 | |
| DT | | | 3.4 | 4.4 | 4.0 | 5.3 | |
| HT | | | 3.9 | 3.8 | n.b. | n.b. | |

Wie aus den Tabellen 6 und 7 hervorgeht, kann durch Lagerung von Glucose-Dehydrogenase bzw. Diaphorase in Gegenwart von carbaNAD über einen Zeitraum von 18 Wochen eine hohe Enzymaktivität aufrecht erhalten werden, während die Abbaurate der Enzyme in Gegenwart von NAD deutlich stärker ausgeprägt ist

### Beispiel 7

Zur Bestimmung des Einflusses von NAD bzw. carbaNAD auf die Thermostabilität verschiedener Dehydrogenasen wurden die Enzyme (1 mg/ml) in einem ersten Schritt für 20 Stunden bei 4°C gegen den jeweiligen Messpuffer dialysiert. Anschließend wurden den Proben 3.8 mM NAD bzw. carbaNAD hinzugefügt und die Proben bei 4°C gehalten.

Um die Bindung von NAD bzw. carbaNAD an die verschiedenen Dehydrogenasen zu bestimmen, wurde eine dynamische Differenzkalorimetrie (DSC) durchgeführt, wobei die kalorimetrischen Scans bei einer Temperatur zwischen 20°C und 100°C und einer Scangeschwindigkeit von 120°C/h vorgenommen wurden und jede Probe in dreifacher Ausführung vermessen wurde.

Die DSC-Vorrichtung wurde vor Durchführung der Messungen gemäß dem MicroCal-Handbuch gereinigt und kalibriert, um einen korrekten Betrieb zu gewährleisten. Als Kontrolle wurde Lysozym (1 mg/ml) in 0.1 M Glycin-HCl, pH 2.4, vor und nach jedem Scanvorgang in doppelter Ausführung vermessen. Messzellen, Ventil und Spritze wurden nach jeweils sechs Injektionen dreimal mit Wasser gewaschen. Die Datenanalyse erfolgte unter Verwendung der MicroClas Origin Software.

Glucose-Dehydrogenase-Mutante2 in Tris-Puffer, pH 8.0, führte sowohl in Abwesenheit als auch in Anwesenheit von NAD zu wohldefinierten Peaks, wobei der Schmelzpunkt (T_{M}) in Abwesenheit von NAD 79.1 °C und in Anwesenheit von NAD 80.8°C betrug. Die Bindung von Glucose-Dehydrogenase-Mutante2 an NAD ergab somit eine Verschiebung von T_{M} um mehr als 1.5°C. Mit Ausnahme von Alkohol-Dehydrogenase wurde bei Inkontaktbringen von Enzym und NAD bzw. carbaNAD eine Erhöhung von T_{M} auch im Falle anderer Dehydrogenasen, beispielsweise im Falle von Wildtyp-Glucose-Dehydrogenase, beobachtet.

Tabelle 8 zeigt die mittels DSC-Analyse für die verschiedenen Dehydrogenasen erhaltenen Daten, wobei jeweils Versuchsbedingungen gewählt wurden, die zu einem einzelnen Peak im DSC-Spektrum führten. Der Effekt der Bindung von NAD bzw. carbaNAD an die Dehydrogenase wurde hierbei jeweils mit Scans verglichen, in welchen kein Coenzym (Ligand) zugegen war. Als Messpuffer diente jeweils 0.1 M Tris-Puffer, pH 8.5, soweit nichts anderes angegeben ist.

**Tabelle 8**

| Dehydrogenase | Ligand | ΔH_{cal} (kcal/mol) | T_{M} (°C) |
|---|---|---|---|
| Alkohol-Dehydrogenase * | ----- | 260 | 55.5 |
| | NAD | 300 | 55.5 |
| | carbaNAD | 310 | 55.4 |
| Glucose-Dehydrogenase (wildtyp) | ----- | 64 | 52.8 |
| | NAD | 110 | 63.5 |
| | carbaNAD | 84 | 58.5 |
| D-Lactat-Dehydrogenase ≠ | ----- | 18 | 58.4 |
| | NAD | 19 | 60.4 |
| | carbaNAD | 19 | 59.1 |
| L-Lactat-Dehydrogenase (aus Schwein) | ----- | 80 | 59.8 |
| | NAD | 85 | 61.8 |
| | carbaNAD | 82 | 61.5 |
| L-Lactat-Dehydrogenase (aus Hase) | ----- | 64 | 58.2 |
| | NAD | 72 | 60.1 |
| | carbaNAD | 66 | 60.2 |
| Malat-Dehydrogenase ‡ | ----- | 310 | 42.4 |
| | NAD | 380 | 45.1 |
| | carbaNAD | 340 | 43.2 |

| | | | |
|---|---|---|---|
| * 0.75 M Glycin-Natriumpyrophosphat, pH 9.0 ≠ 0.1 M Tris-Puffer, pH 7.5 ‡ 0.5 M Carbonatpuffer, pH 10.0 | | | |

## Patentansprüche

1. Verfahren zur Stabilisierung eines Enzyms,
**dadurch gekennzeichnet,**
**dass** man das Enzym in Gegenwart eines stabilisierten Coenzyms lagert, wobei als Enzym eine Alkohol-Dehydrogenase der Nummer EC 1.1.1.2, eine Glucose-6-phosphat-Dehydrogenase der Nummer EC 1.1.1.49 oder eine Diaphorase der Nummer EC 1.6.99.2 verwendet wird.

2. Verfahren nach Anspruch 1,
**dadurch gekennzeichnet,**
**dass** als stabilisiertes Coenzym eine stabilisierte Nikotinamid-Adenin-Dinukleotid-(NAD/NADH)-Verbindung, eine stabilisierte Nikotinamid-Adenin-Dinukleotidphosphat-(NADP/NADPH)-Verbindung oder eine Verbindung der Formel (I) 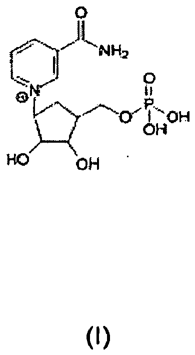 verwendet wird.

3. Verfahren nach Anspruch 1 oder 2,
**dadurch gekennzeichnet,**
**dass** als stabilisiertes Coenzym eine Verbindung der allgemeinen Formel (II) verwendet wird: 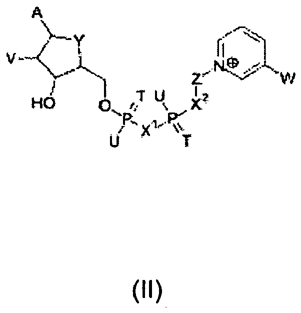 mit
A = Adenin oder ein Analog davon,
T = jeweils unabhängig O, S,
U = jeweils unabhängig OH, SH, BH₃⁻, BCNH₂⁻,
V = jeweils unabhängig OH oder eine Phosphatgruppe, oder zwei Gruppen, die eine cyclische Phosphatgruppe bilden;
W = COOR, CON(R)₂, COR, CSN(R)₂ mit R = jeweils unabhängig H oder C₁-C₂-Alkyl,
X¹, X² = jeweils unabhängig O, CH₂, CHCH₃, C(CH₃)₂, NH, NCH₃,
Y = NH, S, O, CH₂,
Z = ein linearer oder cyclischer organischer Rest ist, mit der Maßgabe, dass Z und der Pyridin-Rest nicht durch eine glycosidische Verbindung verknüpft sind,
oder ein Salz oder gegebenenfalls eine reduzierte Form davon.

4. Verfahren nach Anspruch 3,
**dadurch gekennzeichnet,**
**dass** Z ein gesättiger oder ungesättigter carbocyclischer oder heterocyclischer Fünfring ist, insbesondere eine Verbindung der allgemeinen Formel (III) 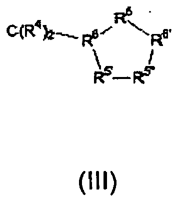 wobei zwischen R⁵' und R⁵" eine Einfach- oder Doppelbindung vorliegen kann, mit
R⁴ = jeweils unabhängig H, F, Cl, CH₃,
R⁵ = CR⁴₂,
R⁵' = O, S, NH, NC₁-C₂-Alkyl, CR⁴₂, CHOH, CHOCH₃, und R⁵" = CR⁴₂, CHOH, CHOCH₃, wenn zwischen R⁵' und R⁵" eine Einfachbindung vorliegt,
R⁵' = R⁵" = CR⁴, wenn zwischen R⁵' und R⁵" eine Doppelbindung vorliegt, und
R⁶, R⁶' = jeweils unabhängig CH oder CCH₃.

5. Verfahren nach einem der Ansprüche 1 bis 4,
**dadurch gekennzeichnet,**
**dass** als stabilisiertes Coenzym carbaNAD verwendet wird.

6. Verfahren nach einem der Ansprüche 1 bis 5,
**dadurch gekennzeichnet,**
**dass** man das stabilisierte Enzym für eine Dauer von mindestens 2 Wochen, bevorzugt von mindestens 4 Wochen, und am stärksten bevorzugt von mindestens 8 Wochen lagert.

7. Verfahren nach einem der Ansprüche 1 bis 6,
**dadurch gekennzeichnet,**
**dass** man das stabilisierte Enzym bei einer Temperatur von mindestens 20°C, bevorzugt von mindestens 25°C, und am stärksten bevorzugt von mindestens 30°C lagert.

8. Verfahren nach einem der Ansprüche 1 bis 7,
**dadurch gekennzeichnet,**
**dass** man das stabilisierte Enzym bei einer relativen Luftfeuchtigkeit von mindestens 50% oder/und in Abwesenheit von Trocknungsmittel lagert.

9. Verfahren nach einem der Ansprüche 1 bis 8,
**dadurch gekennzeichnet,**
**dass** die Lagerung des stabilisierten Enzyms als Trockensubstanz oder in flüssiger Phase erfolgt.

10. Verfahren nach einem der Ansprüche 1 bis 9,
**dadurch gekennzeichnet,**
**dass** man das stabilisierte Enzym in Gegenwart von Licht mit einer Wellenlänge von ≥ 300 nm lagert.

11. Nachweisreagenz zur Bestimmung eines Analyten, welches ein stabilisiertes Enzym enthält, wobei das Enzym mit einem stabilisierten Coenzym stabilisiert ist,
**dadurch gekennzeichnet,**
**dass** es bei einer Lagerung von mindestens 2 Wochen, bevorzugt von mindestens 4 Wochen, und am stärksten bevorzugt von mindestens 8 Wochen bei einer Temperatur von mindestens 20°C, bevorzugt von mindestens 25°C, und am stärksten bevorzugt von mindestens 30°C oder/und in Gegenwart von Licht mit einer Wellenlänge von ≥ 300 nm, gegebenenfalls bei hoher Luftfeuchtigkeit oder/und in Abwesenheit von Trocknungsmittel, eine Abnahme der Enzymaktivität von weniger als 50%, bevorzugt von weniger als 30%, und am stärksten bevorzugt von weniger als 20%, bezogen auf den Ausgangswert der Enzymaktivität, aufweist, wobei das Enzym eine Alkohol-Dehydrogenase der Nummer EC 1.1.1.2, eine Glucose-6-phosphat-Dehydrogenase der Nummer EC 1.1.1.49 oder eine Diaphorase der Nummer EC 1.6.99.2 ist, und wobei das stabilisierte Coenzym eine Verbindung der allgemeinen Formel (II) 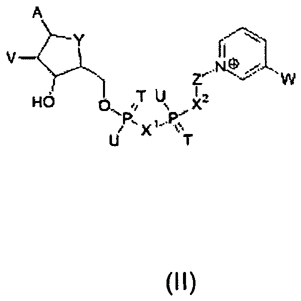 mit
A = Adenin oder ein Analog davon,
T = jeweils unabhängig O, S,
U = jeweils unabhängig OH, SH, BH₃⁻, BCNH₂⁻,
V = jeweils unabhängig OH oder eine Phosphatgruppe, oder zwei Gruppen, die eine cyclische Phosphatgruppe bilden;
W = COOR, CON(R)₂, COR, CSN(R)₂ mit R = jeweils unabhängig H oder C₁-C₂-Alkyl,
X¹, X² = jeweils unabhängig O, CH₂, CHCH₃, C(CH₃)₂, NH, NCH₃,
Y = NH, S, O, CH₂,
Z = ein linearer oder cyclischer organischer Rest ist,
mit der Maßgabe, dass Z und der Pyridin-Rest nicht durch eine glycosidische Verbindung verknüpft sind,
oder ein Salz oder gegebenenfalls eine reduzierte Form davon ist.

12. Nachweisreagenz zur Bestimmung eines Analyten, welches ein stabilisiertes Enzym wie in Anspruch 11 charakterisiert enthält,
**dadurch gekennzeichnet,**
**dass** das stabilisierte Enzym über einen Zeitraum von mindestens 2 Wochen in Gegenwart des stabilisierten Coenzyms gehalten wird, wobei die Lagerung vorzugsweise bei Atmospärendruck, Raumtemperatur (25°C) und einer relativen Luftfeuchtigkeit von mindestens 50% erfolgt.

13. Testelement,
**dadurch gekennzeichnet,**
**dass** es ein Nachweisreagenz nach Anspruch 11 oder 12 enthält.

## Claims

1. Method for stabilising an enzyme,
**characterised in that**
the enzyme is stored in the presence of a stabilised coenzyme, in which an alcohol dehydrogenase of number EC 1.1.1.2, a glucose-6-phosphate dehydrogenase of number EC 1.1.1.49 or a diaphorase of number EC 1.6.99.2 is used as an enzyme.

2. Method according to claim 1,
**characterised in that**
a stabilised nicotinamide adenine dinucleotide (NAD/NADH) compound, a stabilised nicotinamide adenine dinucleotide phosphate (NADP/NADPH) compound or a compound of formula (I) is used as a stabilised coenzyme.

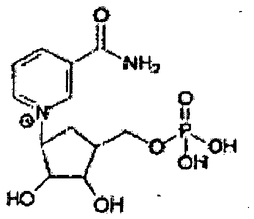

3. Method according to claim 1 or 2,
**characterised in that**
a compound of general formula (II) is used as a stabilised coenzyme: 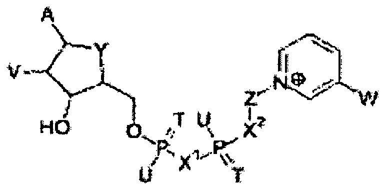 with
A = adenine or an analog of it,
T = independent O, S,
U = independent OH, SH, BH₃⁻, BCNH₂⁻,
V = independent OH or a phosphate group or two groups, which form a cyclic phosphate group;
W = COOR, CON(R)₂, COR, CSN(R)₂ with R = independent H or C₁-C₂-alkyl,
X¹, X² = independent O, CH₂, CHCH₃, C(CH₃)₂, NH, NCH₃,
Y = NH, S, O, CH₂,
Z = a linear or cyclic organic residue,
with the proviso that Z and the pyridine residue are not linked through a glycosidic compound,
or a salt or a reduced form of it if applicable.

4. Method according to claim 3,
**characterised in that**
**Z** is a saturated or unsaturated carbocyclic or heterocyclic five-membered ring, particularly a compound of general formula (III) 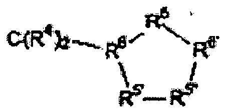 in which there may a single or double bond between R⁵' and R⁵" with
R⁴ = independent H, F, Cl, CH₃,
R⁵ = CR⁴₂,
R⁵' = O, S, NH, NC₁C₂-alkyl, CR⁴₂, CHOH, CHOCH₃ and R⁵" = CR⁴₂, CHOH, CHOCH₃, if there is a single bond between R⁵' and R⁵",
R⁵' = R⁵" = CR⁴ if there is a double bond between R⁵' and R⁵" and
R⁶, R⁶' = independent CH or CCH₃.

5. Method according to one of claims 1 to 4, **characterised in that**
carba-NAD is used as a stabilised enzyme.

6. Method according to one of claims 1 to 5,
**characterised in that**
the stabilised enzyme is stored for a period of at least 2 weeks, preferably at least 4 weeks and most preferably at least 8 weeks.

7. Method according to one of claims 1 to 6,
**characterised in that**
the stabilised enzyme is stored at a temperature of at least 20°C, preferably at least 25°C and most preferably at least 30°C.

8. Method according to one of claims 1 to 7,
**characterised in that**
the stabilised enzyme is stored at a relative humidity of the air of at least 50% and/or in the absence of a drying agent.

9. Method according to one of claims 1 to 8,
**characterised in that**
the stabilised enzyme is stored as a dry substance or in the liquid phase.

10. Method according to one of claims 1 to 9,
**characterised in that**
the stabilised enzyme is stored in the presence of light with a wavelength of ≥ 300 nm.

11. Detection reagent for determining an analyte, which contains a stabilised enzyme, in which the enzyme is stabilised with a stabilised coenzyme,
**characterised in that**
when stored for at least 2 weeks, preferably at least 4 weeks and most preferably at least 8 weeks at a temperature of at least 20°C, preferably at least 25°C and most preferably at least 30°C and/or in the presence of light with a wavelength of ≥ 300 nm with high humidity of the air and/or in the absence of a drying agent if applicable, it shows a reduction in enzyme activity of less than 50%, preferably less than 30% and most preferably less than 20% with reference to the initial value of the enzyme activity, in which the enzyme is an alcohol dehydrogenase of number EC 1.1.1.2, a glucose-6-phosphate dehydrogenase of number EC 1.1.1.49 or a diaphorase of number EC 1.6.99.2 and in which the stabilised coenzyme is a compound of general formula (II) 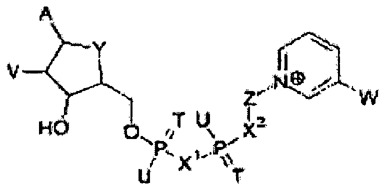 with
A = adenine or an analog of it,
T = independent O, S,
U = independent OH, SH, BH₃⁻, BCNH₂⁻,
V = independent OH or a phosphate group or two groups, which form a cyclic phosphate group;
W = COOR, CON(R)₂, COR, CSN(R)₂ with R = independent H or C₁-C₂-alkyl,
X¹, X² = independent O, CH₂, CHCH₃, C(CH₃)₂, NH, NCH₃,
Y = NH, S, O, CH₂,
Z = a linear or cyclic organic residue, with the proviso that Z and the pyridine residue are not linked through a glycosidic compound, or a salt or a reduced form of it if applicable.

12. Detection reagent for determining an analyte, which contains a stabilised enzyme as **characterised in** claim 11,
**characterised in that**
the stabilised enzyme is kept in the presence of the stabilised coenzyme for a period of at least 2 weeks, in which storage is preferably at atmospheric pressure, room temperature (25°C) and a relative humidity of the air of at least 50%.

13. Test element,
**characterised in that**
it contains a detection reagent according to claim 11 or 12.

## Revendications

1. Procédé pour la stabilisation d'une enzyme, **caractérisé en ce qu'**on stocke l'enzyme en présence d'une coenzyme stabilisée, dans lequel on utilise, à titre d'enzyme, une alcool-déshydrogénase numéro EC 1.1.1.2, une glucose-6-phosphate-déshydrogénase numéro EC 1.1.1.49 ou une diaphorase numéro EC 1.6.99.2.

2. Procédé selon la revendication 1, **caractérisé en ce qu'**on utilise, à titre de coenzyme stabilisée, un composé stabilisé de nicotinamide-adénine-dinucléotide-(NAD/NADH), un composé stabilisé de nicotinamide-adénine-dinucléotidediphosphate-(NADP/NADPH) ou un composé répondant à la formule (I)

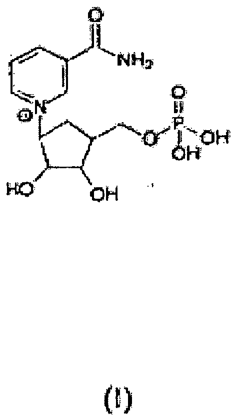

3. Procédé selon la revendication 1 ou 2, **caractérisé en ce qu'**on utilise, à titre de coenzyme stabilisée, un composé répondant à la formule générale (II) : 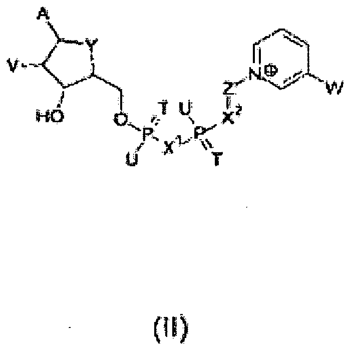 dans laquelle
A représente l'adénine ou un de ses analogues ;
T représente, chaque fois de manière indépendante, un atome d'oxygène, un atome de soufre ;
U représente, chaque fois de manière indépendante, un groupe OH, un groupe SH, un groupe BH₃⁻, un groupe BCNH₂⁻;
V représente, chaque fois de manière indépendante, un groupe OH ou un groupe phosphate, ou bien deux groupes qui forment un groupe phosphate cyclique ;
W représente un groupe COOR, un groupe CON(R)₂, un groupe COR, un groupe CSN(R)₂, dans lesquels R représente, chaque fois de manière indépendante, un atome d'hydrogène ou un groupe alkyle en C₁-C₂ ;
X¹,X² représentent, chaque fois de manière indépendante, un atome d'oxygène, un groupe CH₂, un groupe CHCH₃, un groupe C(CH₃)₂, un groupe NH, un groupe NCH₃ ;
Y représente un groupe NH, un atome de soufre, un atome d'oxygène, un groupe CH₂ ;
Z représente un résidu organique linéaire ou cyclique ; avec cette mesure que Z et le résidu de pyridine ne sont pas reliés par une liaison glycosidique ;
ou un de ses sels ou de manière facultative un de ses dérivés réduits.

4. Procédé selon la revendication 3, **caractérisé en ce que** Z représente un cycle de cinq membres carbocyclique ou hétérocyclique, saturé ou insaturé, en particulier un composé répondant à la formule générale (III) 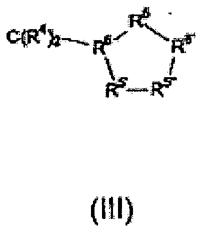 dans laquelle, entre R^{5'} et R^{5"}, une liaison simple ou double peut être présente ;
R⁴ représente, chaque fois de manière indépendante, un atome d'hydrogène, un atome de fluor, un atome de chlore, un groupe CH₃ ;
R⁵ représente un groupe CR⁴₂ ;
R^{5'} représente un atome d'oxygène, un atome de soufre, un groupe NH, un groupe N-alkyle en C₁-C₂, un groupe CR⁴₂, un groupe CHOH, un groupe CHOCH₃, et R^{5"} représente un groupe CR⁴₂, un groupe CHOH, un groupe CHOCH₃, lorsque entre R^{5'} et R^{5"}, une liaison simple est présente ;
R^{5'} est égal à R^{5"} et représente un groupe CR⁴, lorsque entre R^{5'} et R^{5"}, une liaison double est présente ; et
R⁶,R^{6'} représentent, chaque fois de manière indépendante, un groupe CH ou un groupe CCH₃.

5. Procédé selon l'une quelconque des revendications 1 à 4, **caractérisé en ce qu'**on utilise, à titre de coenzyme stabilisée, un groupe carbaNAD.

6. Procédé selon l'une quelconque des revendications 1 à 5, **caractérisé en ce qu'**on stocke l'enzyme stabilisée pendant un laps de temps d'au moins deux semaines, de préférence d'au moins quatre semaines, et de manière la plus préférée d'au moins huit semaines.

7. Procédé selon l'une quelconque des revendications 1 à 6, **caractérisé en ce qu'**on stocke l'enzyme stabilisée à une température d'au moins 20 °C, de préférence d'au moins 25 °C, et de manière la plus préférée d'au moins 30 °C.

8. Procédé selon l'une quelconque des revendications 1 à 7, **caractérisé en ce qu'**on stocke l'enzyme stabilisée sous une humidité atmosphérique relative d'au moins 50 % et/ou en l'absence d'un agent de dessiccation.

9. Procédé selon l'une quelconque des revendications 1 à 8, **caractérisé en ce que** le stockage de l'enzyme stabilisée à lieu sous la forme d'une substance sèche ou en phase liquide.

10. Procédé selon l'une quelconque des revendications 1 à 9, **caractérisé en ce qu'**on stocke l'enzyme stabilisée en présence de lumière à une longueur d'onde ≥ 300 nm.

11. Réactif de décèlement pour la détermination d'un analyte qui contient une enzyme stabilisée, l'enzyme étant stabilisée avec une coenzyme stabilisée, **caractérisé en ce qu'**il présente, lors d'un stockage d'au moins deux semaines, de préférence d'au moins quatre semaines, et de manière la plus préférée d'au moins huit semaines à une température d'au moins 20 °C, de préférence d'au moins 25 °C, et de manière la plus préférée d'au moins 30 °C et/ou en présence de lumière à une longueur d'onde ≥ 300 nm, de manière facultative sous une humidité atmosphérique élevée et/ou en l'absence d'un agent de dessiccation, une diminution de l'activité enzymatique inférieure à 50 %, de préférence inférieure à 30 %, et de manière la plus préférée inférieure à 20 %, rapportés à la valeur de départ de l'activité enzymatique, dans lequel d'enzyme est une alcool-déshydrogénase numéro EC 1.1.1.2, une glucose-6-phosphate-déshydrogénase numéro EC 1.1.1.49 ou une diaphorase numéro EC 1.6.99.2, et dans lequel la coenzyme stabilisée représente un composé répondant à la formule générale (II) : 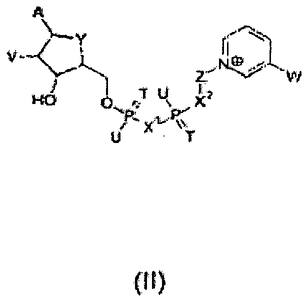 dans laquelle
A représente l'adénine ou un de ses analogues ;
T représente, chaque fois de manière indépendante, un atome d'oxygène, un atome de soufre ;
U représente, chaque fois de manière indépendante, un groupe OH, un groupe SH, un groupe BH₃⁻, un groupe BCNH₂⁻ ;
V représente, chaque fois de manière indépendante, un groupe OH ou un groupe phosphate, ou bien deux groupes qui forment un groupe phosphate cyclique ;
W représente un groupe COOR, un groupe CON(R)₂, un groupe COR, un groupe CSN(R)₂, dans lesquels R représente, chaque fois de manière indépendante, un atome d'hydrogène ou un groupe alkyle en C₁-C₂ ;
X¹,X² représentent, chaque fois de manière indépendante, un atome d'oxygène, un groupe CH₂, un groupe CHCH₃, un groupe C(CH₃)₂, un groupe NH, un groupe NCH₃ ;
Y représente un groupe NH, un atome de soufre, un atome d'oxygène, un groupe CH₂ ;
Z représente un résidu organique linéaire ou cyclique ; avec cette mesure que Z et le résidu de pyridine ne sont pas reliés par une liaison glycosidique ;
ou un de ses sels ou de manière facultative un de ses dérivés réduits.

12. Réactif de décèlement pour la détermination d'un analyte qui contient une enzyme stabilisée telle qu'elle est caractérisée à la revendication 11, **caractérisé en ce qu'**on maintient l'enzyme stabilisée pendant un laps de temps d'au moins deux semaines en présence de la coenzyme stabilisée, dans lequel le stockage a lieu de préférence sous pression atmosphérique, à la température ambiante (25 °C) et sous une humidité atmosphérique relative d'au moins 50 %.

13. Elément de test, **caractérisé en ce qu'**il contient un réactif de décèlement selon la revendication 11 ou 12.
